# EUROPEAN PATENT SPECIFICATION

(11) **EP 2 654 463 B1**
(45) Date of publication and mention of the grant of the patent: **30.05.2018**
(21) Application number: 11820882.6
(22) Date of filing: 21.12.2011
(51) Int. Cl.: A23L 29/00, A23L 33/00, A23L 33/15, A23L 33/12, A61K 9/00, A61K 9/46, A61K 9/14, A61K 9/20

(54) **ANTIOXIDANTS IN FISH OIL POWDER AND TABLETS**
ANTIOXIDANTIEN IN FISCHÖLPULVER UND TABLETTEN
ANTIOXYDANTS DANS LA POUDRE ET LES COMPRIMÉS À BASE D'HUILES DE POISSONS

(30) Priority: 21.12.2010 US 201061425333 P
(43) Date of publication of application: 30.10.2013
(73) Proprietor: Omegatri AS, 0422 Oslo (NO)
(72) Inventor: TORGERSEN, Trine-Lise, N-0666 Oslo (NO); KLAVENESS, Jo, N-1166 Oslo (NO); MYRSET, Astrid, Hilde, N-0873 Oslo (NO)
(74) Representative: Algemeen Octrooi- en Merkenbureau B.V.
(86) International application number: PCT/IB2011/003266
(87) International publication number: WO 2012/085671

(56) References cited:
- WO-A1-91/17670
- WO-A1-2010/035013
- WO-A2-02/43659
- WO-A2-2008/146016
- CH-A- 311 405
- DE-A1- 10 244 907
- US-A1- 2010 272 792

## Description

### Field of the Invention

This invention relates to a stable powder according to the main claim and to an oral delivery vehicle comprising the stable powder. Described is the use of antioxidants to reduce oxidation of powders, tablets, gels and emulsions comprising high concentrations and high doses of omega-3 fatty acids or derivatives thereof.

### Background of the Invention

Omega-3 fatty acids have received considerable interest during the recent years due to the increased awareness about their beneficial effects in a variety of conditions and diseases. The types of omega-3 fatty acids of special relevance are the so called long chain omega-3 fatty acids of marine origin, primarily eicosapentaenoic acid (EPA) and docosahexaenoic acid (DHA). These essential fatty acids cannot be made by the human body, but are available from various marine organisms. They are stored as oils in the fillet of oily fish and in the liver of white fish types like cod. They are key building blocks of human cell membranes and affect as such various cell functions like membrane fluidity, cell to cell signaling, mobility of cells, and formation of secondary signals. They are highly concentrated in the brain and appear to be particularly important for cognitive and behavioural function. They are released from the phospholipid bilayer of cellular membranes in response to stimuli such as wounding, and have key roles in modulation of the immune- and inflammatory response.

Increasing the dietary intake of fish oil is reported to be cardio-protective, antiinflammatory and anti-carcinogenic. They are also important for healthy neurology, psychiatry and vision, and sufficient supply of omega-3 during perinatal life seems to reduce the probability of developing allergy. Omega-3 fatty acids are also reported to have beneficial effects on skin, both due to the effects as a cell membrane constituent as well as to functional roles as e.g. an immune modulator and stimulator of collagen synthesis. Symptoms of omega-3 fatty acid deficiency include extreme tiredness (fatigue), poor memory, heart problems, mood swings or depression, poor circulation and dry skin.

It is very important to maintain a balance between omega-3 and omega-6 (another class of essential fatty acid) in the diet. A healthy diet should consist of roughly one to four times more omega-6 fatty acids than omega-3 fatty acids. With the development of convenience foods and a general decline in the consumption of unprocessed foodstuffs such as fresh fish, fruit and vegetables, the typical American diet tends to contain 11 to 30 times more omega-6 fatty acids than omega-3 fatty acids, and many researchers believe this imbalance is a significant factor in the rising rate of inflammatory disorders in the United States. In contrast, however, the Mediterranean diet consists of a healthier balance between omega-3 and omega-6 fatty acids and many studies have shown that people who follow this diet are less likely to develop heart disease. The Mediterranean diet does not include much meat (which is high in omega-6 fatty acids) and emphasizes foods rich in omega-3 fatty acids including whole grains, fresh fruits and vegetables, fish, olive oil, garlic, as well as moderate wine consumption.

Thus, since their discovery in the 1970s, and as the understanding of the various positive effects of marine omega-3 has increased during the recent years, a variety of omega-3 containing products have appeared on the market. The predominate dosage form for omega-3 fatty acid formulation is currently free oil or capsules containing a considerable amount of oily liquid and therefore delivering a sufficient dose. Other dosage forms such as powders have been developed and are increasingly used, especially as food additives, for omega-3 enriched functional food. A challenge for all dosage forms of omega-3 of marine origin is, however, the fact that these fatty acids are very susceptible towards oxidation, causing unpleasant smell and taste. Oxidised omega-3 can potentially also cause harm to the body. Keeping the omega-3 fresh and non-oxidised is therefore crucial to obtain the maximal beneficial effects, and the skilled man has therefore been looking at ways of preventing oxidation.

Fatty acid based products are used in high doses all over the world both as pharmaceutical products and nutraceutical products, and the use of antioxidants are common in fish oils and fish oil capsules. Tocopherols (vitamin E) and other lipid-soluble derivatives of compounds known for their antioxidative properties, such as ascorbyl palmitate, are commonly used in oils and capsulated oils. For new dosage forms, such as powders and powder based tablets, the antioxidants or antioxidant combinations traditionally used in oils and oil capsules are not necessarily effective, and there is a need to develop antioxidants and antioxidant combinations that are effective in novel dosage forms such as powders and tablets. Effective antioxidants in these types of dosage forms could also be hydrophilic compounds.

Previously, the use of hydrophilic antioxidants such as ascorbic acid or its lipophilic derivative ascorbyl palmitate, alone or in combination with other compounds such as metal chelators or traditional radical scavenger such as tocopherols or natural polyphenol extracts, have been described in some forms of fish oil or fish oil derived products, with varying success with regards to protection against lipid oxidation.

Han et al (1990) described a method for solubilizing ascorbic acid in sardine and soybean oil using reversed micelles. The peroxide value of oil with solubilized ascorbic acid remained low compared to control or oil with solubilized δ-tocopherol or rosemary extract. A synergistic effect was shown for ascorbic acid and δ-tocopherol and for ascorbic acid and rosemary extract in fish oil.

Nishina et al (1991) described the effect of α-, γ- and δ-tocopherol and L-ascorbic acid on ethyl eicosapentanoate (EPA-ethyl ester) and methyl linoleate (ALA methyl ester), by measuring oxygen absorption. An aqueous solution with ascorbic acid was mixed with the lipid solution containing tocopherols by continuous stirring. In methyl linoleate, the ascorbic acid had synergistic effect with the tocopherols. In ethyl eicosapentanoate, there was no effect of ascorbic acid.

Frankel et al (2002) described the effect of EDTA in fish and algal oils, and in emulsions based on these oils. In the oils, EDTA was an efficient antioxidant because of the low iron:EDTA molar concentrations. In the corresponding emulsions, the iron:EDTA molar concentrations were higher, and EDTA exerted pro-oxidative effects. When higher concentrations of EDTA were added, so the molar concentration was higher than that of iron, EDTA was an efficient antioxidant in emulsions as well.

Nielsen et al (2004) described the influence of metal chelators on oxidative stability in oil-in-water emulsions (milk drinks and mayonnaise). EDTA had a strong antioxidative effect in especially mayonnaise, and the effect was concentration dependent.

Olsen et al (2005) described the effect α-tocopherol concentrate and a mixture of α-tocopherol and ascorbyl palmitate on cod liver oil. Sensory analysis, PV-analysis, AnV-analysis and measurement of volatiles by GC-MS were used to assess effects. Ascorbyl palmitate is ascorbic acid esterified with palmitic acid, and is believed to be a more suitable form of adding ascorbic acid to oil samples, as ascorbic acid itself is to hydrophilic to be able to be dissolved in the oil.Where α-tocopherol did not have significant effect on sensory perception or PV, the mixture of TOH and AP had significant effect on both reducing the PV and on improving the sensory properties of the oil. The effects were correlated with changes in the profiles of volatiles in the oil.

Let et al (2005) described antioxidative effects of tocopherols (mix of α- and γ-), ascorbyl palmitate and EDTA in milk emulsions enriched with fish oils. Ascorbyl palmitate almost completely retarded oxidation in the emulsions, while EDTA had no effect. Nor were any interactions between ascorbyl palmitate and EDTA observed.

Olsen et al (2006) described the enrichment of salmon paté with cod liver oil, and the addition of EDTA or citric acid to limit lipid oxidation. EDTA exerted slight effects on sensory properties of the paté, and very little on other parameters used to characterize lipid oxidation.

Let et al (2007) describes the effects of tocopherol, EDTA and ascorbyl palmitate in salad dressing enriched with fish oil. EDTA was the most efficient antioxidant. Ascorbyl palmitate exerted pro-oxidative effects at high concentration in this system, and a weak antioxidative effect at low concentration. Addition of all three compounds simultaneously completely inhibited oxidation. The combination ascorbyl palmitate and EDTA was not studied.

In a review by Jacobsen et al (2008) different antioxidants and antioxidant mixtures in fish oil enriched food systems was evaluated, primarily in oil-in-water (O/W) emulsions such as milk, mayonnaise and salad dressing. Many factors affecting both oxidation and antioxidation were taken into account, including components of the systems, presence of metals, concentration of ingredients and antioxidants, pH, processing factors etc. Several antioxidants were evaluated: EDTA, lactoferrin, tocopherols, ascorbic acid/ascorbyl palmitate, propyl gallate/gallic acid and plant phenolics. EDTA was found to have effect as a metal chelator in systems where metal ion initiation is believed to be of significance for the oxidation process. Ascorbic acid was found to be pro-oxidative in some systems and without effect in others, and ascorbyl palmitate was found to have some effect in some systems. The recommendation from the authors was that ascorbic acid should be avoided in fish oil enriched food emulsions. Synergistic effects were not evaluated.

Haak et al (2009) described the effect of rosemary extract, green tea extract, tocopherol, trolox, ascorbic acid and ascorbyl palmitate in pork patties (meat, not fish). In this system, ascorbic acid was a pro-oxidant, while ascorbyl palmitate had no effect.

WO2010/0305013 discloses compositions in the form of a tablet comprising a krill oil and a carrier and may comprise vitamin C. WO2008/146016 discloses compositions in the form of a tablet comprising fatty acid, vitamins A, C, D and E, and cyclodextrin as a carrier.

Accordingly, the prior art has been unsuccessful in developing lipid products with good stability and shelf-life. What is needed in the art are improved compositions that are stable to oxidation.

### Summary of the Invention

This invention describes antioxidants and combinations of antioxidants used to prevent oxidation of pharmaceutical and nutraceutical products in the form of powders, granulates, tablets, emulsions, gels and the like comprising one or more fatty acids and/or fatty acid derivatives and, optionally, at least one carbohydrate carrier alone or together with vitamins, minerals and/or pharmaceuticals. In particular, the invention concerns the use of antioxidants to reduce oxidation of powders, tablets, gels and emulsions comprising high concentrations and high doses of omega-3 fatty acids or derivatives thereof.

The present invention provides a powder comprising a fatty acid compound, carrier, ascorbic acid and a metal chelator which is EDTA. The powder is capable of maintaining a Totox/kg oil of less than 100, 50 or most preferably 25 for 10, 20, 30, 40 or 50 weeks at room temperature in the presence of oxygen and the absence of light. The ascorbic acid is included at a concentration of 2 mmol - 500 mmol per kg powder, preferably 20-100 mmol per kg powder. In some embodiments, the carrier is selected from the group consisting of cyclodextrin, microcrystalline cellulose, and combinations thereof. In some embodiments, the cyclodextrin is beta-cyclodextrin. In some embodiments, the fatty acid compound is complexed with the cyclodextrin. The metal chelator EDTA is included at a concentration of 10 micromol - 4 mmol per kg powder, preferably 50 micromol - 1.0 mmol per kg powder. In some embodiments, the fatty acid compound is selected from the group consisting of free fatty acids, triglycerides, fatty acid esters, phospholipids and combinations thereof. In some embodiments, the fatty acid compound comprises fatty acid moieties selected from the group consisting of EPA, DHA, and conjugated linoleic acid. In some embodiments, the fatty acid compound comprises a fatty acid compound preparation selected from the group consisting of fish oil, salmon oil, cod liver oil, omega-3 concentrate, krill oil,

and algal oil. In some embodiments, the powder comprises 10 percent to 50 percent fatty acid compound on w/w basis per total mass of powder. In some embodiments, the stable powders further comprise at least a second antioxidant. In some embodiments, the second antioxidant is selected from the group consisting of rosemary extract and cloudberry extract. In some embodiments, the stable powders further comprise a gelling agent.

In some embodiments, the present invention provides a pharmaceutical or nutraceutical tablet for oral administration comprising the stable powder as described above. In some embodiments, the tablet comprises more than 20 wt %, e.g. more than 25 wt %, especially more than 50 wt % of the stable powder. In some embodiments, the tablet comprises more than 120 mg, e.g. at least 200 mg of the fatty acid compound. The tablet may further comprise at least a second active agent. The second active agent is selected from the group consisting of a pharmaceutical, nutraceutical, vitamin, mineral or other health supplementing compound. In some embodiments, the tablet further comprises a functional coating comprising a coating material and at least one functional material. In some embodiments, the coating material is selected from the group consisting of hydroxypropylmethyl cellulose, ethylcellulose, methylcellulose, hypomellose, hydroxyethylcellulose, polyvinylpyrrolidine, polyacrylates, polyethyleneglycol, sugar, gelatin, chitin, chitosan, titanium oxide, pH sensitive polymers and cellulose acetate phthalate. In some embodiments, the functional material is selected from the group consisting of a specifically degradable material, a light absorbing material, a material that enhances hydrophobic stability, and a material that enhances oxidative stability, and combinations thereof. In some embodiments, the specifically degradable material comprises an enzymatically degradable material. In some embodiments, the material that enhances oxidative stability comprises an antioxidant, chelating agent, and combinations thereof. In some embodiments, the tablets further comprise at least one vitamin in addition to ascorbic acid and/or at least one mineral. The tablets may further comprise a disintegrant, excipient, and/or glidant. The tablet core may further comprise at least one additional active agent in addition to the fatty acid compound. The at least one additional active agent may be a pharmaceutical agent or a nutraceutical agent.

In some embodiments, the present invention provides a coated tablet for oral administration comprising a tablet core surrounding by a coating comprising a coating material and at least one functional material. In some embodiments, the coating material is selected from the group consisting of hydroxypropylmethyl cellulose, ethylcellulose, methylcellulose, hypomellose, hydroxyethylcellulose, polyvinylpyrrolidine, polyacrylates, polyethyleneglycol, sugar, gelatin, chitin, chitosan, titanium oxide, pH sensitive polymers and cellulose acetate phthalate. In some embodiments, the functional material is selected from the group consisting of a specifically degradable material, a light absorbing material, a material that enhances hydrophobic stability, and a material that enhances oxidative stability, and combinations thereof. In some embodiments, the specifically degradable material comprises an enzymatically degradable material. In some embodiments, the material that enhances oxidative stability comprises an antioxidant, chelating agent, and combinations thereof. In some embodiments of the disclosure, the tablet core comprises a compressed powder comprising the stable powder described above. In some embodiments of the disclosure, the tablet core further comprises at least one additional active agent in addition to the fatty acid compound. In some embodiments of the disclosure, the at least one additional active agent is a pharmaceutical agent or a nutraceutical agent. In some embodiments of the disclosure the tablet core further comprises at least one vitamin in addition to ascorbic acid and/or at least one mineral. In some embodiments of the disclosure, the tablet core further comprises a disintegrant, excipient, and/or glidant.

In some embodiments, the present disclosure provides compositions comprising ascorbic acid, a metal chelator, and a fatty acid compound preparation selected from the group consisting of a fatty acid compound gel and fatty acid compound emulsion, said ascorbic acid and metal chelator dispersed in said fatty acid compound preparation, said composition characterized in being capable of maintaining a Totox/kg oil of less than 100, 50 or most preferably 25 for 10, 20, 30, 40 or 50 weeks at room temperature in the presence of oxygen and the absence of light. In some embodiments of the invention, the fatty acid compound gel comprises a fatty acid compound dispersed in a gelling agent. In some embodiments, the fatty acid compound emulsion comprises a fatty acid compound oil phase dispersed in an aqueous phase. In some embodiments of the invention, the ascorbic acid is included at a concentration of 2 mmol-500 mmol per kg of said composition. In some embodiments, the EDTA is included at a concentration of 10 micromol - 4 mmol per kg composition. In some embodiments, the fatty acid compound is selected from the group consisting of free fatty acids, triglycerides, fatty acid esters, phospholipids and combinations thereof. In some embodiments, the fatty acid compound comprises fatty acid moieties selected from the group consisting of EPA, DHA, and conjugated linoleic acid. In some embodiments, the fatty acid compound comprises a fatty acid compound preparation selected from the group consisting of fish oil, salmon oil, cod liver oil, omega-3 concentrate, krill oil, and algal oil. In some embodiments, the powder comprises 10 percent to 50 percent fatty acid compound on w/w basis per total mass of powder. In some embodiments, the compositions further comprise at least a second antioxidant. In some embodiments, the second antioxidant is selected from the group consisting of rosemary extract and cloudberry extract.

Described is a pharmaceutical or nutraceutical oral delivery vehicle for oral administration comprising the composition as described above. In some embodiments, the oral delivery vehicle is selected from the group consisting of a tablet and a capsule. The oral delivery vehicle may be chewable. The oral delivery vehicle may further comprise a sweetening. The oral delivery vehicles may further comprise at least a second active agent. The second active agent may be selected from the group consisting of a pharmaceutical, nutraceutical, vitamin, mineral or other health supplementing compound.

Described is the use of the foregoing compositions and oral delivery vehicles for oral administration to a subject and methods comprising administering the compositions and oral delivery vehicles to subjects, preferably to subjects that can benefit from administration of omega-3 fatty acids. In some embodiments of the disclosure, the compositions and oral delivery vehicles are administered to subjects in need of treatment, prevention, or amelioration of a disease or condition selected from the group consisting of obesity, metabolic syndrome, diabetes, high blood triglycerides, high cholesterol, arteriosclerosis, platelet adhesion, platelet aggregation, plaque formation, and inflammation.

Described are foods, food supplements, food products, dietary supplements, nutritional bars, beverages and other functional foods comprising the stable powders and compositions described above.

### Description of the Figures

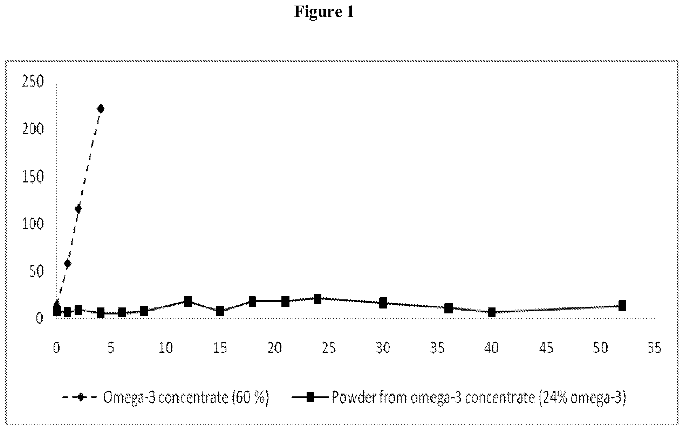

Figure 1 provides a graph of results of an accelerated stability study of a powder and the oil from which is was made, with oxygen exposure at ambient temperature for up to 52 weeks

### Definitions

The "nutraceutical" refers to any substance that is a food or a part of a food and provides medical or health benefits, including the prevention and treatment of disease.

The term "derivative of a fatty acid," e.g. omega-3 or omega-6 fatty acid, is meant a salt, amide or ester thereof, or any other compound where the COOH group is functionalised in such a way that it will return to a COOH group upon treatment, e.g. upon hydrolysis, e.g. a phospholipid thereof. Typically however, the fatty acid compounds in the tablets of the invention are in the form of esters, e.g. C₁₋₁₂-alkyl esters, especially methyl and ethyl esters, or more especially glycerides, in particular triglycerides, i.e. the fatty acid derivative is a triglyceride. Preferred salts are those of alkali metals, e.g. sodium or ammonium salts, in particular polyamino alcohol salts. Mixtures of derivatives and/or acids may be present.

The term "fatty acid compound" is used to cover a fatty acid per se or a derivative thereof.

"Total Oxidation Value (TOTOX)" is used to describe total oxidation to which an oil has been exposed. PV x 2 + AV = TOTOX. Peroxide value (PV) measures primary oxidation. Anisidine value (AV) is a measurement of secondary oxidation. Precisely, it is the measure of aldehyde production during oxidation of fats. For both AV and PV, and hence TOTOX, a lower number is better.

### Detailed Description of the Invention

This invention relates to antioxidants and combinations of antioxidants used to prevent oxidation of pharmaceutical and nutraceutical products in the form of stabilized powders, granulates, tablets, gels and emulsions comprising one or more fatty acids and/or fatty acid derivatives (i.e., fatty acid compounds) and optionally at least one carbohydrate carrier optionally together with vitamins, minerals and/or pharmaceuticals. In particular, the invention concerns the use of antioxidants to reduce oxidation of powders comprising high concentrations and high doses of omega-3 fatty acids or derivatives thereof. As described above, the prior art has generally been unsuccessful in providing omega-3 based products with good oxidation stability.

Surprisingly, the present inventors have now found that ascorbic acid is highly effective in protecting powders, gels and emulsions containing high doses of long-chain polyunsaturated omega-3 fatty acids of marine origin (i.e.,fatty acid compositions) against lipid oxidation, and hence keep the marine fatty acid compounds free from rancidity and subsequent unpleasant odour and taste for a long time. Especially surprising is the fact that ascorbic acid is far more effective in this respect than the lipophilic version of ascorbic acid, ascorbyl palmitate. This effect is further enhanced in combinations of ascorbic acid with other compounds, preferably metal chelators and radical scavengers.

Accordingly, the present invention provides stable fatty acid powders comprising fatty acid compound(s) that are prone to oxidation. The stable fatty acid powders comprise a fatty acid compound, carrier, ascorbic acid and a metal chelator which is EDTA. The ascorbic acid is included in the powder at a concentration of 2mmol - 500 mmol per kg powder, preferably at a concentration of about 10 - 100 mmol per kg fatty acid powder. The stable fatty acid powders further comprise the metal chelator EDTA (ethylenediaminetetraacetic acid) at a concentration of from 10 micromol - 4 mmol per kg powder, preferably about 50 micromol - 1.0 mmol per kg powder.

The stable fatty acid powders of the present invention demonstrate exceptional long term stability. The stable fatty acid powders of the present invention have the property of being oxidatively stable as measured by the Totox index (See Figure 1). The stable fatty acid powders of the present invention are capable of a Totox/kg oil in the powder of less than 100, 50 or most preferably 25 Totox/kg oil in the powder for greater than or at least 10, 20, 30, 40 or 50 weeks at room temperature in the presence of oxygen and the absence of light.

The stable fatty acid powders of the present invention may comprise one or more antioxidants in addition to the ascorbic acid. Preferred additional antioxidants include plant extracts such as rosemary extract, cloudberry extract, tea extract, flavonoids, tocopherol, tochopherol, BRT, BRA, Butyl hydroxy anisol (BHA), Butyl hydroxy toluene (BHT), propyl gallate, octyl gallate, and any of the GRINDOX™ series antioxidants.

The present invention provides a stable fatty acid powder comprising a fatty acid compound, carrier and ascorbic acid and a metal chelator which is EDTA. In some preferred embodiments, the fatty acid composition is a powder that is white and free flowing. In some embodiments, the powder comprises at least 10, 20, 30, 40 or up to 50 percent fatty acid compound on a w/w basis (mass lipid as a percent of total mass of powder). In some preferred embodiments, the carrier is beta-cyclodextrin. In some especially preferred embodiments, the carrier and fatty acid compound form a complex where the fatty acid compound is absorbed by the beta-cyclodextrin.

The fatty acid compounds of the present invention comprise one or more fatty acid derivatives. Fatty acids or derivatives thereof present in the powders of the invention are preferably unsaturated, especially polyunsaturated. Most preferably, the powder of the invention comprise at least one omega-3 fatty acid compound.

Any fatty acid compound, preferably an omega-3 fatty acid compound, present in the powder can preferably be synthetic or semisynthetic but preferably it is derived from a natural source such as a plant oil or an animal oil. Oils which contain fatty acids, typically present as esters of the fatty acids, are well known in the art. Suitable plant oils include rapeseed oil, corn oil, soya oil, sunflower oil, vegetable oil and olive oil. The natural source of the fatty acid may also be an animal oil such as tallow oil.

In some preferred embodiments, the source of the fatty acid compound is a marine oil, such as a fish oil or krill oil. Crude marine oil used in this invention can be derived from any marine source such as fish, especially seawater fish such as tuna, sardines, salmon, mackerel, herring, trout, halibut, cod, haddock, catfish, sole etc. The use of oily fish is preferred. In some embodiments, the oil is derived from krill. In some embodiments, the crude marine oil will derive from marine mammals such as seals, walrus or sea lions, preferably seals. Seal oil has been found to be especially rich in omega-3 fatty acid compounds, e.g. of the order of 20-25 wt % and therefore forms an ideal starting material to form the tablets of the invention. Seal oils are available from a variety of commercial sources.

The fatty acid powders can contain one fatty acid compound or a mixture of fatty acid compounds. Preferably, it contains a mixture of fatty acid compounds, especially unsaturated fatty acid compounds, especially a mixture of polyunsaturated fatty acid compounds. It will be appreciated that the fatty acid compositions of the invention might also contain saturated fatty acid compounds as these are also present in naturally occurring unsaturated fatty acid compound sources.

An unsaturated fatty acid compound contains one or more carbon carbon double bonds in the carbon backbone. Preferably, the carbon backbone is polyunsaturated. Preferably, at least one fatty acid is an omega-3 fatty acid compound in which the double bond most distant from the carboxylic acid functionality is located at the third bond counted from the end (omega) of the carbon chain. The fatty acid compound may also be an omega-6 fatty acid compound where the double bond most distant from the carboxylic acid functionality is located at the sixth bond counted from the end (omega) of the carbon chain. A powder of the invention most preferably contains a variety of omega-3 and also some omega-6 fatty acid compounds.

The total concentration of omega-3 fatty acid compounds in a crude oil varies depending on the natural source in question but, for example, in sea fish, the amount of the omega-3 compounds is approximately 20-35 wt %.

Unsaturated fatty acid compounds which can form part of the powder of the invention may be those of formula (I):

CH₃(CH₂)ₙ=(CH=CH--CH₂)ₘ--(CH₂)ₛ--C-OOH (I)

wherein n, m and s are integers, e.g. of 1 to 10;
or a derivative thereof.

Subscript n is preferably 1. Subscript m is preferably 2 to 8. Subscript s is preferably 1 to 6. Ideally, the carbon chain is linear although it is within the scope of the invention for the backbone to carry alkyl side chains such as methyl or ethyl. (For this formula DHA n=1, m=6 and s=1, for EPA n=1, m=5 and s=1. In ALA, n=4, m=2 and s=6).

Omega-3 fatty acid compounds of use in the powders of the invention are preferably those which contain at least 18 carbon atoms in the carbon backbone. Lower chain fatty acids (those of 17 carbon atoms or less in the backbone) appear to show fewer useful therapeutic effects, but can be useful in applications like fish or animal feed.

Thus, preferred unsaturated fatty acid compounds are those of formula (I')

CH₃CH₂CH=CH--R--COOH (I')

wherein R is a C₁₃+ alkylene group (e.g. C₁₃₋₂₅) optionally containing 1 or more double bonds, preferably non-conjugated;
or a derivative thereof.

Ideally, the R group is linear although it is within the scope of the invention for the backbone to carry alkyl side chains such as methyl or ethyl. The total number of carbon atoms in the chain is preferably 16 to 22. Moreover, R is preferably 13, 15, 17, 19 etc. i.e. the number of carbon atoms in the chain is preferably even. Whilst it will be appreciated that the omega-3 enriched powders of the invention will, most likely, contain a variety of different omega-3 based compounds, highly preferred compounds of formula (I) are eicosapentaenoic acid (EPA) and docosahexaenoic acid (DHA) or derivatives thereof, e.g. triglyceride, phospholipid, sodium salt or polyamino alcohol salt thereof.

In a highly preferred embodiment, the fatty acid compounds comprise a mixture of DHA and EPA or derivatives thereof. The ratio of such compounds may be 30:70 to 70:30, preferably 40:60 to 60:40 EPA/DHA. The most mixtures of compounds are mixtures comprising at least EPA and DHA in the form of free acids, physiologically acceptable salts, ethyl esters, phospholipids and triglycerides.

The powders of the invention may also contain omega-6 fatty acids. Preferred omega-6 fatty acids are those of formula (II):

CH₃CH₂CH₂CH₂CH₂CH=CH--R"--COOH (II)

wherein R" is a C₅+ alkylene group (e.g. C₁₀₋₂₂) optionally containing 1 or more double bonds; or derivatives thereof.

In some preferred embodiments, the R" group is linear although it is within the scope of the invention for the backbone to carry alkyl side chains such as methyl or ethyl.

The number of carbon atoms in R" is preferably 10, 12, 14, 16 etc., i.e. the number of carbon atoms in the chain is preferably even. In a preferred embodiment the omega-6 fatty acid compound is ALA, gamma-linolenic acid (GLA) or conjugated linoleic acid (CLA), or a derivative thereof, e.g. a triglyceride, phospholipid, sodium salt or polyamino alcohol salt thereof.

Whilst it will be appreciated that the fatty acid compositions of the invention will, most likely, contain a variety of different omega-3 and -6 based compounds, highly preferred compounds of formula (II) are C18, C20 and C22 compounds.

The weight ratio of omega-3 to omega-6 fatty acid compounds in the fatty acid component of the fatty acid compositions of the invention may be of the order 1:1 to 100:1.

Preferably, the fatty acids of the invention will have at least 10 carbon atoms, e.g. at least 12 carbon atoms, such as at least 14 carbon atoms in the fatty acid portion of the molecule, i.e. a fatty acid must comprise at least 10 carbon atoms.

In preferred embodiments, compounds of formula (I), (I') or (II) will be multiply unsaturated, e.g. contain 2 to 10 double bonds, especially 4 to 7 double bonds. Preferably double bonds are not conjugated either to each other or to the carbonyl functionality. At least one, e.g. 2 or 3, preferably all double bonds are preferably in the cis configuration.

Crude oils contain a variety of fatty acids or derivatives thereof (e.g. esters thereof, in particular triglycerides) having differing carbon chain lengths and differing levels of unsaturation. Of course not all these fatty acids will be omega-3 unsaturated fatty acid compounds, some will be omega-6 unsaturated, some may be saturated oils. Fatty acid compositions comprising a mixture of these fatty acid compounds are therefore encompassed by certain embodiments of the present invention.

One preferred aspect of the present invention relates to stabilized fatty acid powders comprising concentrated fatty acid compounds, comprising for example primarily EPA or DHA ethyl ester or triglyceride. Whatever the nature of the fatty acid material, it is readily available from commercial sources. Concentrated DHA and EPA can be purchased and converted to an appropriate derivative using known processes.

In some embodiments, the stabilized fatty acid compositions of the invention may contain at least 10 wt % fatty acid compound (in total), e.g. at least 20 wt % or at least 25 wt % or at least 30 wt % or at least 40 wt % such as at least 50 wt % fatty acid compound (in total).

The present invention provides stabilized fatty acid powders. In some preferred embodiments, the stabilized powders comprise at least one fatty acid or derivative thereof (especially omega-3 and/or omega-6 fatty acid(s) or esters) and cyclodextrin, especially in the form of a fatty acid compound cyclodextrin complex. Any form of cyclodextrin may be used in the invention, e.g. alpha, beta or gamma cyclodextrin. These are commercially available materials. The stable powders of the present invention preferably comprise a complex between the fatty acid compound and the carrier. In some embodiments, the powders of the present invention are directly compressable and/or tabletable, with or without additional carriers or excipients. The powders preferably have the property of retaining the lipid without expression of the fatty acid compound from the tablet following compression. In some embodiments, the powders of the present invention are characterized in that the fatty acid compound-carrier complex does not comprise microencapsulated particles comprising, for example, microdroplets formed from phospholipid emulsions. In contrast, the fatty acid compound-carrier complexes of the present invention are preferably characterized by being nanoscale in size.

The term "complex" is used here to designate that fatty acid compound is associated with a carrier, e.g., cyclodextrin or microcrystalline cellulose, through some form of intermolecular non-covalent bonds. These bonds include normally relative weak bonds like hydrophobic interactions. When the carrier is cyclodextrin, the fatty acid in the complex is normally located within the core of the cyclodextrin molecule but could also be associated with other parts of the molecule. The most preferred size of the cyclodextrins are alpha-cyclodextrin, beta-cyclodextrin and gamma-cyclodextrin. Cyclodextrins with different cavity size can optionally be substituted. The preferred substituent include alkyl groups, hydroxyalkyl groups, acyl groups. For reviews on pharmaceutical acceptable cyclodextrin derivatives see: K. Uekama et al in J. Inclution Phenomena and Macrocyclic Chemistry (2006) 56:page 3-8, T. Loftsson et al. in Am. J. Drug Deliv. (2004)2:page 261-275 and J. Szejtli in J. Inclution Phenomena and Macrocyclic Chemistry (2005)52:1-11. The most preferred cyclodextrins according to the present invention is unsubstituted alpha-, beta- or gamma-cyclodextrins and methyl or hydroxypropyl derivatives thereof. The even most preferred cyclodextrins are beta-cyclodextrin and hydroxypropyl-cyclodextrin. The term "stabilized complex" refers to complexes as just described that are stabilized with ascorbic acid, and more preferably with ascorbic acid and a metal chelator.

The weight ratio between fatty acid compound (or compounds total) and carrier can vary over wide limits. The weight ratio may be in the range of 1:10 to 10:1 (between fatty acid compound (or compounds total) and, e.g., cyclodextrin), such as 1:5 to 5:1, preferably 1:2 to 2:1. In some embodiments the ratio between fatty acid compound (or compounds total) and carrier (e.g., beta-cyclodextrin) is from about 1:5 to 2:3.

A convenient method for forming stabilized complexes of the present invention involves the use of water as a solvent for the cyclodextrin and ascorbic acid which can then be mixed with the fatty acids e.g. in the form of an ester. The complex forms and can be separated, e.g. by filtration and washed. In some embodiments, the beta cyclodextrin is mixed with water and antioxidants to a slurry, and then the fatty acid compound (e.g., fish oil) is mixed in by a kneading process to a soft ice to drinking yogurt texture.

Thus, the stabilized complexes of the present invention can be prepared using state of the art techniques for preparation of cyclodextrin complexes. Typical methods include for example formation of the complex in water, in mixture of water and organic solvents or water-free organic solvents at ambient temperatures. Typical organic solvents include methanol, ethanol, isopropanol, acetone, DMSO, DMF and acetonitrile. The ratio between cyclodextrin and fatty acid compound should preferably be low, typically below 1. The cyclodextrin complex with fatty acid compound and ascorbic acid is isolated by filtration, evaporation or freeze drying.

The stabilized complex of fatty acid compound with cyclodextrin and ascorbic acid is a solid, preferably a stable powder. It should not be an oily material. It will be appreciated that sometimes to achieve a stable powder a solid may need to be ground. In a further preferred embodiment therefore the stabilized complex will be suitable for grinding to form a stable powder.

Drying of the stabilized complexes can be carried out by any known means. The material can be vacuum dried or simply left to dry in a nitrogen atmosphere. It could be gently heated to encourage drying. Preferred drying methods do, however, include freeze drying and spray drying, included spray granulation. Spray drying techniques are disclosed in "Spray Drying Handbook", K. Masters, 5th edition, Longman Scientific Technical UK, 1991, the disclosure of which is hereby incorporated by reference at least for its teaching of spray drying methods.

It will be appreciated that where there is more than one fatty acid compound present, there can be more than one cyclodextrin complex formed. It is also within the scope of the invention for a mixture of cyclodextrins to be used, e.g. beta and gamma cyclodextrin or derivatives thereof. The most preferred combinations include EPA/DHA ethyl ester/cyclodextrin mixtures.

The stable powders described above are preferably used for the manufacture of the tablets of the invention. The tablets of the invention preferably comprise a stable fatty acid compound/cyclodextrin/ascorbic acid complex where the composition weight is more than 50%, such as greater than 80%. In some embodiments, the complex can form more than 90% of the tablet weight, more preferably more than 95% of the tablet weight, and most preferably more than 99% of the tablet weight. The tablets of the invention preferably contain at least 100 mg, e.g. at least 125 mg, preferably at least 150 mg, such as at least 200 mg, e.g. from about 200 to about 400 mg.

In order to form tablets it is highly preferred if the fatty acid compound is in the form of a solid, especially a stable powder, especially a crystalline solid. This can be achieved through complex formation as described above or achieved by isolating a fatty acid compound in solid form.

The tablets of the invention may be produced by compression or compaction of a formulation containing stable powder and certain excipients, typically selected to aid in the processing and to improve the properties of the tablet. The tablets of the invention may be coated or uncoated and can be made from powdered, crystalline materials. Tablets may be plain, film or sugar coated, bisected, embossed, layered, or sustained release. Any film coating preferably comprise of a physiologically acceptable water-soluble organic polymer. They can be made in a variety of sizes, shapes and colours.

Excipients which may be present include diluents, binders, disintegrants, lubricants, glidants and in many cases, colorants. The excipients used are classified according to the function they perform. For example, a glidant may be used to improve the flow of powder blend in the hopper and into the tablet die.

Lubricants are typically added to prevent the tableting materials from sticking to punches, minimize friction during tablet compression, and allow for removal of the compressed tablet from the die. Such lubricants are commonly included in the final tablet mix in amounts usually less than 1% by weight. The most commonly used lubricants are magnesium stearate, stearic acid, hydrogenated oil, and sodium stearylfumarate. Anti adherents like talc can preferably be added to prevent the tableting materials from sticking to punches.

Tablets often contain diluents, such as lactose, which are added to increase the bulk weight of the blend resulting in a practical size for compression. This is often necessary where the dose of the drug is relatively small so the use of diluents is favoured in this invention where high doses of the fatty acid compounds are required. Typical diluents include for example dicalcium phosphate, calcium sulphate, lactose, cellulose, kaolin, mannitol, sodium chloride, dry starch and other sugars. The cellulose can preferably be microcrystalline cellulose (Avicel).

Binders are agents which impart cohesive qualities to the powdered material. Commonly used binders include starch, gelatin, sugars such as sucrose, glucose, dextrose, and lactose, natural and synthetic gums, carboxymethylcellulose, methylcellulose, polyvinylpyrrolidone, ethylcellulose and waxes.

Disintegrants are often included to ensure that the tablet has an acceptable rate of disintegration. Typical disintegrants include starch derivatives, crospovidone, croscaramelose and salts of carboxymethylcellulose. Some binders, such as starch and cellulose, are also excellent disintegrants.

Other desirable characteristics of excipients include high compressibility to allow strong tablets to be made at low compression forces, good flow properties that can improve the flow of other excipients in the formula and cohesiveness (to prevent tablet from crumbling during processing, shipping and handling). The skilled man knows the type of excipients appropriate for tablet formulation.

It is preferred if the total weight of excipients (i.e., excipients other than the carrier used to make the powder such a beta-cyclodextrin or microcrystalline cellulose) in a tablet of the invention is no more than 20 wt % of that tablet, preferably less than 15 wt % of the tablet, especially less than 10 wt % of the tablet.

The three processes for making compressed tablets are wet granulation, direct compression, and dry granulation (slugging or roller compaction). Whilst all three methods can be used to form the tablets of the invention, it is preferred if direct compression is employed.

Dry granulation consists of blending, slugging the ingredients, dry screening, lubrication, and compression. The wet granulation method is used to convert a powder mixture into granules having suitable flow and cohesive properties for tableting. The procedure consists of mixing the powders in a suitable blender followed by adding the granulating solution under shear to the mixed powders to obtain a granulation. The damp mass is then screened through a suitable screen and dried by tray drying or fluidized bed drying. Alternately, the wet mass may be dried and passed through a mill. The overall process includes: weighing, dry powder blending, wet granulating, drying, milling, blending lubrication and compression.

Direct compression is a relatively quick process where the powdered materials are compressed directly without changing the physical and chemical properties of the drug. The fatty acid compound, direct compression excipients and any other auxiliary substances, such as a glidant and lubricant are blended, e.g. in a twin shell blender or similar low shear apparatus before being compressed into tablets.

The advantages of direct compression include uniformity of blend, few manufacturing steps involved, (i.e. the overall process involves weighing of powders, blending and compression, hence less cost), elimination of heat and moisture, prime particle dissociation, and physical stability.

However, direct compression is usually limited to those situations where the drug or active ingredient has a crystalline structure and physical characteristics required to form pharmaceutically acceptable tablets. Since the fatty acid compounds of the invention typically present as oils, the use of direct compression to form oral dosage forms of fatty acid compounds is not reported. Moreover, since excipients need to be added to a direct compression formulation to allow the compression process to take place manufacturers are often limited to using the direct compression method in formulations containing a low dose of the active ingredient per compressed tablet as otherwise tablet sizes become to large for swallowing.

A solid dosage form containing a high dose drug (i.e. where the drug itself comprises a substantial portion of the total compressed tablet weight) can only be directly compressed if the drug itself has sufficient physical characteristics (e.g. cohesiveness) for the ingredients to be directly compressed. Surprisingly, the inventors have found that fatty acid compounds and complexes of the invention possess the necessary physical characteristics. The fatty acid compounds and complexes of the invention have unexpectedly good flow and compression characteristics. The material, optionally mixed with excipients as described above, for example microcrystalline cellulose, talc and magnesium stearate, is free-flowing and sufficiently cohesive to act as a binder.

In some preferred embodiments, the fatty acid compounds which can be presented in solid form, especially as cyclodextrin complexes, especially complexes comprising high amounts of fatty acid compound, can be tabletted without prior granulation (i.e. by direct compression). The most preferred method of production of tablets of the invention is therefore by direct compression.

The size of the tablets, according to the present invention can vary. The tablet diameter can vary from 6 mm to 20 mm, preferably 8 to 14 mm. The tablet weight can vary from 100 mg to 3 grams. The most preferred tablets have tablet weights between 200 mg and 1 gram with a diameter from 10 to 15 mm.

The tablets are for oral administration either by direct swallowing thereof or by any other known means, e.g. chewable tablets, dissolution or suspension of the tablet in a drinkable liquid and so on.

Whilst the tablets are primarily for use with human consumers, tablets might also be administered to animals, especially mammals, e.g. higher mammals.

The fatty acid compounds can be dispersed in an emulsion, preferably a stabilized emulsion comprising the water soluble antioxidants disclosed herein.
The emulsions can be oil in water (O/W) emulsions. By this it is meant that they contain a continuous aqueous phase and a discontinuous lipophilic phase, i.e. a fatty acid compound phase. The oil droplets are preferably of micrometer size or smaller, e.g. with mode droplet diameters in the range 50 to 100000 nm, preferably 100 to 50000 nm, especially 160 to 6000 nm, more especially 500 to 6000 nm The emulsions can be sterile and pyrogen-free. Sterile emulsions may be prepared using sterile components under sterile conditions. Alternatively the oil phase may be prepared, sterilized, then emulsified under sterile conditions with a sterile aqueous phase. As a further alternative, the emulsion may be produced and then sterilized, e.g. by heat treatment (e.g. autoclaving), by irradiation (e.g. gamma-irradiation) or, where the oil droplet size is small, by sterile filtration. The emulsions may contain further components, e.g. stabilizers, antioxidants, viscosity modifiers, vitamins, minerals, pH adjusting agents, plasma anions (e.g. Na⁺, Ca²⁺, K⁺, especially Na⁺ and Ca²⁺, optionally deriving from their chloride salts), emulsifiers, etc.

The emulsions can be administered orally and can be are incorporated into gels as described in more detail below. Where the emulsion is to be administered parenterally, it preferably has an aqueous phase which has a tonicity which is within 20% of isotonicity, more preferably within 10% of isotonicity, especially within 2% of isotonicity. Isotonicity in this regard may be taken to be 300 mOsm/kg for human subjects.
This may be achieved particularly conveniently by use of physiologically tolerable salts (e.g. chloride salts) of plasma anions such as Na⁺, Ca²⁺ and K⁺. Sodium salts and combinations of sodium and calcium salts are preferred; however non-ionic agents such as glycerol or sugars may be used to increase tonicity. Formulation in this manner has a cardioprotective effect which is especially important for aged or reduced immune function patients.

Examples of emulsifying agents that may be used include amphiphilic compounds such as phospholipids (e.g. lecithin), polyoxyethylene sorbates (Tweens), sorbitan carboxylic acid esters (Spans), polyalkyleneoxides (e.g. PEGs and Pluronics). Such emulsifiers are preferably used in the preparation of the emulsions of the invention and desirably are present in the emulsions in amounts of 0.02 to 10% wt relative to total emulsion weight. pH modifying agents may be included in the compositions if this does not prejudice the stability of the pharmaceutical agent. Generally the aqueous phase of the emulsions should have a pH in the range 4.5 to 7.5, especially 5.5. to 7.0.

The emulsions preferably contain 5 to 300 mg/mL of a fatty acid compound, preferably marine fatty acid compounds or omega-3 fatty acid compounds, e.g. 50 to 250 mg/mL, especially 100 to 200 mg/mL and preferably have viscosities at 20 C. of 100 mPas or less. However, the emulsions may be prepared in concentrate, rather than ready-to-use, form and thus may contain for example up to 400 mg/mL omega-3 fatty acid compounds.

The fatty acid compounds can be dispersed in a gel, preferably a stabilized gel comprising the water soluble antioxidants disclosed herein. The gelling agent used in the aqueous phase of the emulsion may be any physiologically tolerable gelling agent (preferably a saccharide (e.g. an oligosaccharide or polysaccharide), a protein or a glycoprotein) or combination capable of forming a soft, chewable, self-supporting gelled oil-in-water emulsion. Many such materials are known from the food and pharmaceutical industry and are discussed for example in Handbook of hydrocolloids, G O Phillips and P A Williams (Eds.), Woodhead Publishing, Cambridge, UK, 2000. The gelling agents are preferably materials capable of undergoing a sol-gel transformation, e.g. under the influence of a change in physiochemical parameters such as temperature, pH, presence of metal ions (e.g. group 1 or 2 metal ions), etc. Preferred gelling agents include gelatins, alginates and carrageenans. However, the use of gelatins is especially preferred as breakdown in the throat of trapped fragments is ensured and as cores having the desired properties may readily be produced using gelatins.

The fatty acids compounds can be dispersed in an emulsion which includes the gelling agent. Gelatins having an imino acid content of 5 to 25% wt. are preferred, more especially those having an imino acid content of 10 to 25% wt. The gelatins will typically have a weight average molecular weight in the range 10 to 250 kDa, preferably 75 to 220 kDa, especially 80 to 200 kDa. Gelatins having no Bloom value or low Bloom values of 60-300, especially 90-200 are preferred. Where a gelatin of no Bloom value, e.g. a cold water fish gelatin, is used, this will typically be used together with another gelatin or other gelling agent. The combination of cold water and warm water fish gelatins is especially preferred. The gelatin will typically be present in the aqueous phase at a concentration of 1 to 50% wt., preferably 2 to 35% wt., particularly 5 to 25% wt. In the case of mixtures of gelatin and polysaccharides, the weight ratio of gelatin to polysaccharide in the aqueous phase will typically be 50:1 to 5:1, preferably 40:1 to 9:1, especially 20:1 to 10:1. The pH of the aqueous phase of the emulsion is preferably in the range 2 to 9, particularly 3 to 7.5. The aqueous phase preferably has a gelling temperature in the range 10 to 30 C., more preferably 15 to 28 C., and a melting temperature in the range 20 to 80.degree. C., more preferably 24 to 60 C., especially 28 to 50 C.

Where a sweetener is included in the aqueous phase, this will typically be selected from natural sweeteners such as sucrose, fructose, glucose, reduced glucose, maltose, xylitol, maltitol, sorbitol, mannitol, lactitol, isomalt, erythritol, polyglycitol, polyglucitol and glycerol and artificial sweeteners such as aspartame, acesulfame-K, neotame, saccharine, sucralose. The use of non-carciogenic sweeteners is preferred and the use of xylitol is especially preferred.

The stabilized fatty acid gels and emulsions may be provided in an oral delivery vehicle such as a tablet, capsule (e.g., gel capsule), syrup, or other solid or liquid dosage form, including gel boluses. In particularly preferred embodiments, the oral delivery vehicle is preferably a soft, chewable solid or gel. The oral delivery vehicle may preferably be further formulated or coated as described elsewhere herein.

In a further preferred aspect of the disclosure, the fatty acid powders can be formulated together with other active agents. Active agents which could be combined with the complexes of the invention include pharmaceuticals, nutraceuticals, vitamins, minerals and other health supplementing compounds. Combination with drugs is highly preferable.

The most preferred drugs to be formulated together with fatty acid compounds in tablets are drugs for treatment and/or prophylaxis of diseases in the cardiovascular system and in bone. Typical such drugs include ACE-inhibitors; like for example enalapril, angiotensin II receptor antagonists like losartan, beta-blockers like propranolol, plasma cholesterol reducing compounds like statins, typically simvastatin or atorvastatin, and bisphosphonates like for example alendronate. Other favourable drugs include glucosamine.

Highly preferred additional components in the powders of the invention also include simvastatin, atorvastatin, glucosamine, vitamins and/or minerals in particular calcium.
also described within the disclosure are tablets comprising fatty acid powders of the invention together with these nutraceutical or pharmaceutical ingredients. Typical nutraceutical ingredients can be calcium, or other minerals, water-soluble vitamins like Vitamin B or Vitamin C, lipid-soluble vitamins like Vitamin A, D, K (e.g. K2) or E and ingredients present in the nature like for example berrys, herbs and extracts thereof.

It is also possible for an additional ingredient present in the powders of the disclosure to be present as a stabilized cyclodextrin complex, especially where this is a vitamin, especially vitamin K2 and most especially MK-7. This forms a still yet further aspect of the invention which therefore provides a stabilized complex formed between vitamin K2 and cyclodextrin, in particular a complex formed between MK-7 and cyclodextrin, especially a pharmaceutical or nutraceutical tablet for oral administration comprising such a complex. These complexes can also be combined with fatty acid/cyclodextrin complexes to form especially preferred tablets of the invention.

The powders of the disclosure may also contain folic acid and other well known over the counter health supplements such as echinacea.

In some embodiments, the present invention provides core structures such as tablets, powders, granulates, microparticles or nanoparticles that are coated with a functional coating. The functional coating preferably comprises at least one functional material in addition to a coating material. The functional coating can be used for protection of tablets with any tablet core prone to oxidationThe coating can be used for protection of tablets with any omega-3 containing tablet core against oxidation. The functional coating can be used to coat tablets formed from the stable powders described above.

The term "functional coating" as used herein refers to a coating with a function different from standard coatings like film coating (smoothening the tablet surface to improve swallowing) and enteric coating (release of tablet content in the intestine and not in the stomach). The functional coating can serve several functions, e.g., delivery of active pharmaceutical ingredient (API) or the nutraceutical ingredient (NI), improvement of photostability of the API or NI, improvement of the hydrophilic stability of API or NI, improvement of oxidative stability of the API or NI, as well as other functions related the stability, use or delivery.

The functional coating can aid in the delivery an API or NI. The coating material can for example be used to secure specific delivery of API or NI. A coating material that is degraded in the lower part of the gastrointestinal system can be used for colon specific delivery. coating material can be utilized that is enzymatically broken down in the colon or in the lower part of the small intestine by specific enzymes present in the lower part of the gastrointestinal system.These functional coatings an be useful for delivery of colon specific photodynamic agents for diagnosis and/or treatment. Examples of materials to be used in the functional coating include, but are not limited to, 5-ALA and 5-ALA esters.

The functional coating can provide improvement of photostability of the API or NI. The coating material can, for example, be a coating comprising a coloured substance that absorbs light and thereby improve the stability for any light-sensitive APIs or NIs. Several compounds are light sensitive; including omega-3 comprising oils, powders and tablets. The coloured substance can for example be a dark green, blue or black substance.

The functional coating can provide for improvement of the hydrophilic stability of API or NI. The coating material can be any material that keep the water away from the API or NI in the tablet core, the powder or the granulate. The coating material can be a material that reacts with water and/or a material that physically keep the water away from the API and/or the NI.

The functional coating can provide for improvement of oxidative stability of the API or NI. Several APIs and Nls are sensitive to oxidation. For example, intelligent packaging that keeps air (oxygen) away from the products will improve the oxidative stability. Accordingly, the tablets, powders or granulates can be packaged with a material that is impervious to oxygen, e.g, blister packaging, foil packaging and other packing systems well known for other products. A coating that protects against oxidation can be one that physically keeps oxygen away from the API or NI.Such coating materials can form a gas gas-tight or oxygen-tight membrane around the material and includes materials well known for coating of nutraceutical and pharmaceutical products. The functional coating material can be a material that protects against oxidation by reaction with oxygen oxidized species. This type of coating chemically neutralizes the oxidation process. Examples of such materials include, but are not limited to, materials that easily react with oxygen, oxygen radicals or other compounds generated by oxygen. Such compounds include, but are not limited to, antioxidants, chelating agents and other compounds that are known to improve the oxidative stability. Typical antioxidants include any physiologically acceptable antioxidant; natural synthetic and semisynthetic.A mixture of antioxidants can be utilized. One or more antioxidants are combined with EDTA or more metal chelators. Examples of antioxidants useful in the functional coating of the present invention include, but are not limited to, rosemary extract, cloudberry extract, tea extract, flavonoids, tocopherol, tochopherol, BRT, BRA, Butyl hydroxy anisol (BHA), Butyl hydroxy toluene (BHT), propyl gallate, octyl gallate, and any of the GRINDOX™ series antioxidants.

In some embodiments, the antioxidant, ascorbic acid is included in the powder at a concentration of 2mmol-500 mmol per kg coating, more preferably at a concentration of 20-100 mmol per kg coating. In some embodiments, EDTA is included in the coating at a concentration of from 10 micromol - 4 mmol per kg coating, more preferably 50 micro mol - 1.0 mmol per kg coating. In some preferred embodiments, the antioxidants are combined with coating materials as described in more detail below. Coatings that improve the oxidative stability can be used to improve the stability of any oxidatively sensitive API and NI, for example, oil and powders comprising omega-3 fatty acid compounds as described above.

As is apparent, the present invention provides a particulate formulation such as a tablet, powder or granulate comprising a core coated with a functional coating. The coating applied on the cores may in principle be any coating such as, e.g, a film coating, a sugar coating, a bioadhesive coating, or a so-called modified release coating, to which a further functional material such as an antioxidant is added. The coating provides e.g. the desired release profile of the active substance included in the cores or, alternatively, masks the taste of bad-tasting active substances, e.g. bitter tasting active substances such as, e.g., noscapine or theophylline. In some cases, the cores according to the invention may contain two or more layers of coating e.g. a first coating which governs the release rate ofthe active substance and a second layer which is bioadhesive. Either later may comprise the further functional coating material such as an antioxidant.

The formulations can be designed to release the active substance substantially immediately upon administration or at any suitable time or time period after administration. The latter type of formulations is generally known as modified release formulations.

In accordance with the United States Pharmacopoeia, the term "modified release dosage forms" includes two types of dosage forms, namely "extended-release" dosage forms and "delayed-release" dosage form. An extended-release dosage form is defined as one that allows at least a two-fold reduction in dosing frequency as compared to that drug presented as a conventional dosage form (i.e. as a solution or a prompt drug-releasing conventional solid dosage form). A delayed-release dosage form is defined as one that releases a drug (or drugs) at a time other than promptly after administration. Enteric coated formulations are delayed release dosage forms.

In the present context, the term "modified release formulation" embraces the above mentioned "extended-release" and "delayed-release" dosage forms and, accordingly, the following types offormulation are also included in the definition of the term "modified release formulation": i) formulations which create a substantially constant concentration of the active substance within the body over an extended period of time, ii) formulations which after a predetermined lag time create a substantially constant concentration of the active substance within the body over an extended period of time, iii) formulations which sustain the action of the active substance (such as a drug substance) during a predetermined time period by maintaining a relatively constant, effective drug level in the body and at the same time minimizing the incidence of undesirable side effects associated with fluctuations in the plasma level of the active substance (sawtooth kinetic pattern), iv) formulations which attempt to localise drug action by, e.g., spatial placement of a modified release formulation adjacent to or in the diseases tissue or organ, v) formulations which attempt to target drug action by using carriers or chemical derivatives to deliver the active substance to a particular adjacent to or in the diseases tissue or organ, v) formulations which attempt to target drug action by using carriers or chemical derivatives to deliver the active substance to a particular target cell type, and vi) formulations which are coated with an enteric coating ("gastro-resistant", "enterosoluble", "entero-coated", or simply "enteric" formulations).

Modified release formulations may also be denoted "extended release", "delayed release", "controlled release", "sustained release", "prolonged release", "programmed release", "timerelease", "rate-controlled", and/or "targeted release" formulations.

A suitable coating may, for example be, a functional material such as an antioxidant in conjunction with: a film coating, e.g. a coating based on one or more of the material selected from the following: polyvinyl-alcohols, hydroxypropyl-methylcellulose, ethylcellulose, methylcellulose, hydroxyethylmethylcellulose, hydroxypropylcellulose, carboxymethylcellulose sodium, acrylate polymers (such as, e.g. Eudragit™), polyethylene glycols and polyvinylpyrrolidone; a sugar coating; a bioadhesive coating, such as, e.g., a coating comprising a bioadhesive substance such as, e.g., a fatty acid ester such as, e.g., fatty acid esters wherein the fatty acid component of the fatty acid ester is a saturated or unsaturated fatty acid having a total number of carbon atoms of from Cs to C₂₂; specific examples are glyceryl monooleate, glyceryl monolinoleate, glycerol monolinolenate, or mixtures thereof, a modified release coating, such as, e.g., an enteric coating, e.g. a coating which is such that when the coated cores is swallowed by a human or an animal, it will be substantially unaffected by the chemical, enzymatic and other conditions prevailing within the stomach during passage through this part of the digestive system, but will substantially dissolve or otherwise disintegrate within the intestinal tract of the human or animal in question, thereby releasing the active substance within the intestines. An enteric coating may be based on one or more ofthe material selected from the following: methacrylic acid copolymers (e.g. Eudragit™ L or S), cellulose acetate phthalate, ethylcellulose, hydroxypropylmethylcellulose acetate succinate, polyvinyl acetate phthalate, and shellac; or a modified release coating, e.g. a coating based on one or more materials selected from the following: shellac; waxes such as, e.g., beeswax, glycowax, castor wax, carnauba wax; hydrogenated oils such as, e.g., hydrogenated castor oil, hydrogenated coconut oil, hydrogenated rape seed oil, hydrogenated soyabean oil; fatty acid or fatty alcohol derivatives such as, e.g, stearyl alcohol, glyceryl monostearate, glyceryl distearate, glycerol palmitostearate; acrylic polymers such as, e.g., acrylic resins (Eudragit™ RL and RS, acrylic resins are copolymers of acrylic and methacrylic acid esters with a low content of quaternary ammonium groups) poly(methyl methacrylate), methacrylate hydrogels, ethylene glycol methacrylate; polylactide derivatives such as, e.g., dl-polylactic acid, polylacticglycolic acid copolymer; cellulose derivatives, such as, e.g., ethylcellulose, cellulose acetate, cellulose propionate, cellulose butyrate, cellulose valerate, cellulose acetate propionate, cellulose acetate butyrate; vinyl polymers such as, e.g., polyvinyl acetate, polyvinyl formal, polyvinyl butyryl, vinyl chloride-vinyl acetate copolymer, ethylene-vinyl acetate copolymer, vinyl chloride-propylene-vinyl acetate copolymer, polyvinylpyrrolidone; glycols such as, e.g., 1,3-butylene glycol, polyethylene glycols; polyethylene; polyester; polybutadiene; and other high molecular synthetic polymers. In some embodiments, the coating of the present invention comprises copolymers of the above mentioned substances, grafted or mixed chemically or physically. Preferred copolymers include, but are not limited, copolymers of polyvinyl alcohol and PEG.

The coating material may be admixed with various excipients such as, e.g., plasticizers; antiadhesives such as, e.g., colloidal silicium dioxide (fumed silica), talc, and magnesium stearate; colorants; flavours; and solvents in a manner known per se. Examples ofplasticizers for use in accordance with the invention include polyhydric alcohols such as, e.g., propylene glycol, glycerol, and polyethylene glycol; acetate esters such as, e.g., glyceryl triacetate (Triacetin), triethyl acetate, and acetyl triethyl acetate; phthalate esters such as, e.g., diethylphthalate; glycerides such as, e.g., acetylated monoglycerides; oils such as, e.g., castor oil, mineral oil, and fractionated coconot oil; and dibutyl sebacate.

The coating is applied on the cores from a solution and/or suspension in an organic solvent or in an aqueous medium. Employment of an aqueous medium is preferred due to safety, economy and environment. Examples of suitable organic solvents for use in coating the cores are alcohols such as, e.g., methanol, ethanol, isopropanol, and propanol; ketones such as, e.g. acetone, and toluene; esters such as, e.g., ethyl acetate, and ethyl lactate; chlorinated hydrocarbons such as, e.g. methylene chloride, and I: I :I trichloroethane.

The application of the coating may be performed in a fluidized bed but any suitable coating apparatus may be applied such as those well known by a person skilled in the art (e.g. pan coating, spray-drying, electrostatic coating etc.). When the cores are coated in a fluidized bed apparatus it has proved advantageous to apply the coating composition from a nozzle positioned in the bottom of the fluid bed apparatus, i.e. having the flow of the liquid (the coating composition) and the fluidizing air in a mixed flow except when the coating is performed with a fat or a wax. By using a mixed flow it has shown possible to coat relatively small particles without agglomeration. The amount of coating applied on the cores depends inter alia on the size of the cores, the type of coating employed, the type of the active substance employed, and the desired release pattern.

Described are core structures such as tablets, powders, granulates, microparticles or nanoparticles that are coated with a functional coating. The functional coating preferably comprises at least one functional material in addition to a coating material. The functional coating can be used for protection of tablets with any tablet core prone to oxidation. The coating can be used for protection of tablets with any omega-3 containing tablet core against oxidation. The functional coating is used to coat tablets formed from the stable powders described above.

The term "functional coating" as used herein refers to a coating with a function different from standard coatings like film coating (smoothening the tablet surface to improve swallowing) and enteric coating (release of tablet content in the intestine and not in the stomach). The functional coating can serve several functions, e.g., delivery of active pharmaceutical ingredient (API) or the nutraceutical ingredient (NI), improvement of photostability of the API or NI, improvement of the hydrophilic stability of API or NI, improvement of oxidative stability of the API or NI, as well as other functions related the stability, use or delivery.

The functional coating can aid in the delivery an API or NI. The coating material can for example be used to secure specific delivery of API or NI. In some embodiments, a coating material that is degraded in the lower part of the gastrointestinal system is used for colon specific delivery. Coating material can be utilized that is enzymatically broken down in the colon or in the lower part of the small intestine by specific enzymes present in the lower part ofthe gastrointestinal system.These functional coatings can be useful for delivery of colon specific photodynamic agents for diagnosis and/or treatment. Examples of materials to be used in the functional coating include, but are not limited to, 5-ALA and 5-ALA esters.

The functional coating can provide improvement of photostability of the API or NI. The coating material can, for example, be a coating comprising a coloured substance that absorbs light and thereby improve the stability for any light-sensitive APIs or NIs. Several compounds are light sensitive; including omega-3 comprising oils, powders and tablets. The coloured substance can for example be a dark green, blue or black substance.

The functional coating can provide for improvement of the hydrophilic stability of API or NI. The coating material can be any material that keep the water away from the API or NI in the tablet core, the powder or the granulate. The coating material can be a material that reacts with water and/or a material that physically keep the water away from the API and/or the NI.

The functional coating can provide for improvement of oxidative stability of the API or NI. Several APIs and Nls are sensitive to oxidation. For example, intelligent packaging that keeps air (oxygen) away from the products will improve the oxidative stability. Accordingly, the tablets, powders or granulates can be packaged with a material that is impervious to oxygen, e.g, blister packaging, foil packaging and other packing systems well known for other products. A coating that protects against oxidation can be one that physically keeps oxygen away from the API or NI. Such coating materials form a gas gas-tight or oxygen-tight membrane around the material and include materials well known for coating of nutraceutical and pharmaceutical productsThe functional coating material can be a material that that protects against oxidation by reaction with oxygen or oxidized species. This type of coating chemically neutralizes the oxidation process. Examples of such materials include, but are not limited to, materials that easily react with oxygen, oxygen radicals or other compounds generated by oxygen. Such compounds include, but are not limited to, antioxidants, chelating agents and other compounds that are known to improve the oxidative stability. Typical antioxidants include any physiologically acceptable antioxidant; natural synthetic and semisynthetic. A mixture of antioxidants can be utilizedOne or more antioxidants can be combined with one or more metal chelators such as EDTA. Examples of antioxidants useful in the functional include, but are not limited to, tochopherol, ascorbic acid, BRT and BRA. In some embodiments, the antioxidant, e.g., ascorbic acid is included in the powder at a concentration of 2mmol-1-500 mmol per kg coating, more preferably at a concentration of 20-100 mmol per kg coating. A metal chelator, e.g., EDTA, can be included in the coating at a concentration of from 10 micro mol - 4 mmol per kg coating, more preferably 50 micromol - 1.0 mmol per kg coating. The antioxidants can be combined with coating materials as described in more detail below. Coatings that improve the oxidative stability can be used to improve the stability of any oxidatively sensitive API and NI, for example, oil and powders comprising omega-3 fatty acid compounds as described above.

Described is a particulate formulation such as a tablet, powder or granulate comprising a core coated with a functional coating. The coating applied on the cores may in principle be any coating such as, e.g, a film coating, a sugar coating, a bioadhesive coating, or a so-called modified release coating, to which a further functional material such as an antioxidant is added. The coating provides e.g. the desired release profile of the active substance included in the cores or, alternatively, masks the taste of bad-tasting active substances, e.g. bitter tasting active substances such as, e.g., noscapine or theophylline. In some cases, the cores according to the invention may contain two or more layers of coating e.g. a first coating which governs the release rate ofthe active substance and a second layer which is bioadhesive. Either later may comprise the further functional coating material such as an antioxidant.

The formulations may be designed to release the active substance substantially immediately upon administration or at any suitable time or time period after administration. The latter type of formulations is generally known as modified release formulations.

In accordance with the United States Pharmacopoeia, the term "modified release dosage forms" includes two types of dosage forms, namely "extended-release" dosage forms and "delayed-release" dosage form. An extended-release dosage form is defined as one that allows at least a two-fold reduction in dosing frequency as compared to that drug presented as a conventional dosage form (i.e. as a solution or a prompt drug-releasing conventional solid dosage form). A delayed-release dosage form is defined as one that releases a drug (or drugs) at a time other than promptly after administration. Enteric coated formulations are delayed release dosage forms.

In the present context, the term "modified release formulation" embraces the above mentioned "extended-release" and "delayed-release" dosage forms and, accordingly, the following types of formulation are also included in the definition of the term "modified release formulation": i) formulations which create a substantially constant concentration of the active substance within the body over an extended period of time, ii) formulations which after a predetermined lag time create a substantially constant concentration of the active substance within the body over an extended period of time, iii) formulations which sustain the action of the active substance (such as a drug substance) during a predetermined time period by maintaining a relatively constant, effective drug level in the body and at the same time minimizing the incidence of undesirable side effects associated with fluctuations in the plasma level of the active substance (sawtooth kinetic pattern), iv) formulations which attempt to localise drug action by, e.g., spatial placement of a modified release formulation adjacent to or in the diseases tissue or organ, v) formulations which attempt to target drug action by using carriers or chemical derivatives to deliver the active substance to a particular target cell type, and vi) formulations which are coated with an enteric coating (" gastro-resistant", "enterosoluble", "entero-coated", or simply "enteric" formulations).

Modified release formulations may also be denoted "extended release", "delayed release", "controlled release", "sustained release", "prolonged release", "programmed release", "time release", "rate-controlled", and/or "targeted release" formulations.

A suitable coating for a formulation may, for example be, a functional material such as an antioxidant in conjunction with: a film coating, e.g. a coating based on one or more of the material selected from the following: hydroxypropyl-methylcellulose, ethylcellulose, methylcellulose, hydroxyethylmethylcellulose, hydroxypropylcellulose, carboxymethylcellulose sodium, acrylate polymers (such as, e.g. Eudragit™), polyethylene glycols and polyvinylpyrrolidone; a sugar coating; a bioadhesive coating, such as, e.g., a coating comprising a bioadhesive substance such as, e.g., a fatty acid ester such as, e.g., fatty acid esters wherein the fatty acid component of the fatty acid ester is a saturated or unsaturated fatty acid having a total number of carbon atoms of from Cs to C₂₂; specific examples are glyceryl monooleate, glyceryl monolinoleate, glycerol monolinolenate, or mixtures thereof, a modified release coating, such as, e.g., an enteric coating, e.g. a coating which is such that when the coated cores is swallowed by a human or an animal, it will be substantially unaffected by the chemical, enzymatic and other conditions prevailing within the stomach during passage through this part of the digestive system, but will substantially dissolve or otherwise disintegrate within the intestinal tract of the human or animal in question, thereby releasing the active substance within the intestines. An enteric coating may be based on one or more ofthe material selected from the following: methacrylic acid copolymers (e.g. Eudragit™ L or S), cellulose acetate phthalate, ethylcellulose, hydroxypropylmethylcellulose acetate succinate, polyvinyl acetate phthalate, and shellac; or a modified release coating, e.g. a coating based on one or more materials selected from the following: shellac; waxes such as, e.g., beeswax, glycowax, castor wax, carnauba wax; hydrogenated oils such as, e.g., hydrogenated castor oil, hydrogenated coconut oil, hydrogenated rape seed oil, hydrogenated soyabean oil; fatty acid or fatty alcohol derivatives such as, e.g, stearyl alcohol, glyceryl monostearate, glyceryl distearate, glycerol palmitostearate; acrylic polymers such as, e.g., acrylic resins (Eudragit™ RL and RS, acrylic resins are copolymers of acrylic and methacrylic acid esters with a low content of quaternary ammonium groups) poly(methyl methacrylate), methacrylate hydrogels, ethylene glycol methacrylate; polylactide derivatives such as, e.g., dl-polylactic acid, polylacticglycolic acid copolymer; cellulose derivatives, such as, e.g., ethylcellulose, cellulose acetate, cellulose propionate, cellulose butyrate, cellulose valerate, cellulose acetate propionate, cellulose acetate butyrate; vinyl polymers such as, e.g., polyvinyl acetate, polyvinyl formal, polyvinyl butyryl, vinyl chloride-vinyl acetate copolymer, ethylene-vinyl acetate copolymer, vinyl chloride-propylene-vinyl acetate copolymer, polyvinylpyrrolidone; glycols such as, e.g., 1,3-butylene glycol, polyethylene glycols; polyethylene; polyester; polybutadiene; and other high molecular synthetic polymers.

The coating material may be admixed with various excipients such as, e.g., plasticizers; antiadhesives such as, e.g., colloidal silicium dioxide (fumed silica), talc, and magnesium stearate; colourants; and solvents in a manner known per se. Examples ofplasticizers for use in accordance with the invention include polyhydric alcohols such as, e.g., propylene glycol, glycerol, and polyethylene glycol; acetate esters such as, e.g., glyceryl triacetate (Triacetin), triethyl acetate, and acetyl triethyl acetate; phthalate esters such as, e.g., diethylphthalate; glycerides such as, e.g., acetylated monoglycerides; oils such as, e.g., castor oil, mineral oil, and fractionated coconot oil; and dibutyl sebacate.

The coating is applied on the cores from a solution and/or suspension in an organic solvent or in an aqueous medium. Employment of an aqueous medium is preferred due to safety, economy and environment. Examples of suitable organic solvents for use in coating the cores are alcohols such as, e.g., methanol, ethanol, isopropanol, and propanol; ketones such as, e.g. acetone, and toluene; esters such as, e.g., ethyl acetate, and ethyl lactate; chlorinated hydrocarbons such as, e.g. methylene chloride, and I: I :I trichloroethane.

The application of the coating may be performed in a fluidized bed but any suitable coating apparatus may be applied such as those well known by a person skilled in the art (e.g. pan coating, spray-drying, electrostatic coating etc.). When the cores are coated in a fluidized bed apparatus it has proved advantageous to apply the coating composition from a nozzle positioned in the bottom of the fluid bed apparatus, i.e. having the flow of the liquid (the coating composition) and the fluidizing air in a mixed flow except when the coating is performed with a fat or a wax. By using a mixed flow it has shown possible to coat relatively small particles without agglomeration. The amount of coating applied on the cores depends inter alia on the size of the cores, the type of coating employed, the type of the active substance employed, and the desired release pattern.

The health benefits of the fatty acid powders of the invention have been confirmed in many studies. Polyunsaturated fatty acids have been found to keep serum cholesterol levels low, stabilise irregular heartbeat, reduce blood pressure, improve autoimmune disease, improve depression disorders, treat psoriasis, treat rheumatoid arthritis, and to prevent colon cancer. They are generally applied in cardiovascular disorders and for the treatment of bone disorders. The tablets of the invention are of particular interest in the treatment or prevention of hypertriglyceridemia and cardiac infection. Hypertriglyceridemia is a medical condition characterized by increased plasma concentration of triglycerides.

### Experimental

### Example 1 - comparative

### Slurry comprising omega-3 form EPA-ethyl ester and β-cyclodextrin

β-cyclodextrin (700 gram) and water (630 mL) were mixed in a kneader for 5 minutes. EPA-ethyl ester (300 gram) (Epax, Norway) was added and mixed in the kneader for 10 minutes. The oil: β-cyclodextrin ratio was 3:7.

### Example 2 - comparative

### Powder comprising omega-3 and ascorbic acid (low concentration)

To 36.2 gram of the slurry prepared in example 1, 300 µL 0.5 M ascorbic acid_{(aq)} was stirred in with a magnetic stirrer. The product was dried by lyophilisation. The powder contained 50µmol ascorbic acid per gram oil.

### Example 3 - comparative

### Powder comprising omega-3 and CaNa₂EDTA

To 36.2 gram of the slurry prepared in example 1, 75 µL 5 mM CaNa₂EDTA_{(aq)} was stirred in with a magnetic stirrer. The product was dried by lyophilisation. The powder contained 125 nmol CaNa₂EDTA per gram oil.

### Example 4 - comparative

### Powder comprising omega-3 and rosemary extract

To 36.2 gram of the slurry prepared in example 1, 75 mg rosemary extract (Guardian™ Rosemary extract 11, Danisco, Denmark) was stirred in with a magnetic stirrer. The product was dried by lyophilisation. The powder contained 25 mg rosemary extract per gram oil.

### Example 5

### Powder comprising omega-3, ascorbic acid and CaNa₂EDTA (low concentrations)

To 36.2 gram of the slurry prepared in example 1, 300 µL 0.5 M ascorbic acid_{(aq)} and 75 µL 5 mM CaNa₂EDTA_{(aq)} was stirred in with a magnetic stirrer. The product was dried by lyophilisation. The powder contained 50 µmol ascorbic acid and 125 nmol CaNa₂EDTA per gram oil.

### Example 6 - comparative

### Powder comprising omega-3, ascorbic acid and rosemary extract

To 36.2 gram of the slurry prepared in example 1, 300 µL 0.5 M ascorbic acid_{(aq)} and 75 mg rosemary extract (Guardian™ Rosemary extract 11, Danisco, Denmark) was stirred in with a magnetic stirrer. The product was dried by lyophilisation. The powder contained 50 µmol ascorbic acid and 25 mg rosemary extract per gram oil.

### Example 7 - comparative

### Powder comprising omega-3, EDTA and rosemary extract

To 36.2 gram of the slurry prepared in example 1, 75 µL 5 mM CaNa₂EDTA_{(aq)} and 75 mg rosemary extract (Guardian™ Rosemary extract 11, Danisco, Denmark) was stirred in with a magnetic stirrer. The product was dried by lyophilisation. The powder contained 125 nmol CaNa₂EDTA and 25 mg rosemary extract per gram oil.

### Example 8

### Powder comprising omega-3, ascorbic acid, EDTA and rosemary extract (low concentrations)

To 36.2 gram of the slurry prepared in example 1, 300 µL 0.2 M ascorbic acid_{(aq)}, 75 µL 2 mM CaNa₂EDTA_{(aq)} and 30 mg rosemary extract (Guardian™ Rosemary extract 11, Danisco, Denmark) was stirred in with a magnetic stirrer. The product was dried by lyophilisation. The powder contained 20 µmol ascorbic acid, 50 nmol CaNa₂EDTA and 10 mg rosemary extract per gram oil.

### Example 9

### Powder from EPA-ethyl ester oil containing ascorbic acid, EDTA and rosemary extract (high concentrations)

To 36.2 gram of the slurry prepared in example 1, 300 µL 0.8 M ascorbic acid_{(aq)}, 75 µL 8 mM CaNa₂EDTA_{(aq)} and 120 mg rosemary extract (Guardian™ Rosemary extract 11, Danisco, Denmark) was stirred in with a magnetic stirrer. The product was dried by lyophilisation. The powder contained 80 µmol ascorbic acid, 200 nmol CaNa₂EDTA and 40 mg rosemary extract per gram oil.

### Example 10 -comparative

### Slurry comprising omega-3 from cod liver oil and β-cyclodextrin

β-cyclodextrin (150 gram) and water (52.5 mL) were mixed in a mortar grinder for 10 minutes. Cod liver oil (35 gram) (Mollers Tran, Axellus, Norway) was added and mixed in the mortar grinder for 10 minutes. The oil:β-cyclodextrin ratio was 1:3.

### Example 11 - comparative

### Powder comprising omega-3 and ascorbic acid (high concentration)

To 40 gram of the slurry prepared in example 10, 10 mL 1 M ascorbic acid_{(aq)} was added and mixed in with a glass rod. The product was dried in vacuum at room temperature. The powder contained 1375 µmol ascorbic acid per gram oil.

### Example 12

### Powder comprising omega-3, ascorbic acid and CaNa₂EDTA (high concentrations)

To 40 gram of the slurry prepared in example 10, 10 mL 1 M ascorbic acid_{(aq)} and 2 mL 10 mM CaNa₂EDTA_{(aq)} was added and mixed in with a glass rod. The product was dried in vacuum at room temperature. The powder contained 1375 µmol ascorbic acid and 2750 nmol CaNa₂EDTA per gram oil.

### Example 13 - comparative

### Preparation of cloudberry extract

Cloudberries (*Rubus chamaemorus*) (220 g) was homogenized in a mortar grinder for 15 minutes. 200 grams of the homogenate was extracted with 690 mL methanol and 1.7 mL concentrated acetic acid with a magnetic for 5 minutes, sonication for 5 minutes and stirring with a magnet for 15 minutes. After centrifugation at 3000 rmp for 5 minutes the supernatant was filtered and the solvent evaporated in vacuo. The resulting syrup was diluted to 200 mL with water.

### Example 14 - comparative

### Slurry comprising omega-3 from cod liver oil and β-cyclodextrin

β-cyclodextrin (525 gram) and water (375 mL) were mixed in a kneader for 10 minutes. Cod liver oil (225 gram) (Denomega, Norway) was added and mixed in the mortar grinder for 10 minutes. The oil:β-cyclodextrin ratio was 3:7.

### Example 15 - comparative

### Powder comprising omega-3 and quercetin

To 150 gram of the slurry prepared in example 14, 6 mL from a 2.5 mM suspension of quercetin in water was added and stirred in with a glass rod. The product was dried by lyophilisation. The powder contained 500 nmol quercetin per gram oil.

### Example 16 - comparative

### Powder comprising omega-3 and citric acid

To 150 gram of the slurry prepared in example 14, 300 µL 10 mM citric acid_{(aq)} was added and stirred in with a glass rod. The product was dried by lyophilisation. The powder contained 100 nmol citric acid per gram oil.

### Example 17 - comparative

### Powder comprising omega-3, ascorbic acid and quercetin

To 150 gram of the slurry prepared in example 14, 3 mL 1M ascorbic acid_{(aq)} and 6 mL from a 2.5 mM suspension of quercetin in water were added and stirred in with a glass rod. The product was dried by lyophilisation. The powder contained 100 µmol ascorbic acid and 500 nmol quercetin per gram oil.

### Example 18 - comparative

### Powder comprising omega-3, ascorbic acid and citric acid

To 150 gram of the slurry prepared in example 14, 3 mL 1M ascorbic acid_{(aq)} and 300 µL 10 mM citric acid_{(aq)} were added and stirred in with a glass rod. The product was dried by lyophilisation. The powder contained 100 µmol ascorbic acid and 100 nmol citric acid per gram oil.

### Example 19 - comparative

### Powder comprising omega-3, ascorbic acid, quercetin and citric acid

To 150 gram of the slurry prepared in example 14, 3 mL 1M ascorbic acid_{(aq)}, 6 mL from a 2.5 mM suspension of quercetin in water and 300 µL 10 mM citric acid_{(aq)} were added and stirred in with a glass rod. The product was dried by lyophilisation. The powder contained 100 µmol ascorbic acid, 500 nmol quercetin and 100 nmol citric acid per gram oil.

### Example 20

### Powder from cod liver oil containing ascorbic acid, quercetin and EDTA

To 150 gram of the slurry prepared in example 14, 3 mL 1M ascorbic acid_{(aq)}, 6 mL from a 2.5 mM suspension of quercetin in water and 750 µL 10 mM CaNa₂EDTA_{(aq)} were added and stirred in with a glass rod. The product was dried by lyophilisation. The powder contained 100 µmol ascorbic acid, 500 nmol quercetin and 250 nmol CaNa₂EDTA per gram oil.

### Example 21 - comparative

### Slurry comprising omega-3 from salmon oil and β-cyclodextrin

β-cyclodextrin (210 gram) and water (147 mL) were mixed in a mortar grinder for 20 minutes. Salmon oil (70 gram) (Xalar, Marine Harvest, Norway) was added and mixed in the mortar grinder for 10 minutes. The oil:β-cyclodextrin ratio was 1:3

### Example 22 - comparative

### Powder comprising omega-3, ascorbic acid (low concentration) and cloudberry extract (high concentration)

To 20 g of the slurry prepared in example 21, 721 µL 1 M ascorbic acid_{(aq)} and 904 µl of the cloudberry extract prepared in example 16 were added and stirred in using a glass rod. The product was dried by lyophilisation. The powder contained 220 µmol ascorbic acid and 275 µL cloudberry extract per gram oil.

### Example 23 - comparative

### Powder comprising omega-3, EDTA (low concentrations) and cloudberry extract (high concentration)

To 20 g of the slurry prepared in example 21, 357 µL 10 mM CaNa₂EDTA_{(aq)} and 904 µl of the cloudberry extract prepared in example 16 were added and stirred in using a glass rod. The product was dried by lyophilisation. The powder contained 358 µmol CaNa₂EDTA and 275 µL cloudberry extract per gram oil.

### Example 24

### Powder comprising omega-3, ascorbic acid, EDTA and cloudberry extract (low concentrations)

To 20 g of the slurry prepared in example 21, 292 µL 1 M ascorbic acid_{(aq)}, 145 µl 0 mM CaNa₂EDTA and 366 µl of the cloudberry extract prepared in example 16 were added and stirred in using a glass rod. The product was dried by lyophilisation. The powder contained 89 µmol ascorbic acid, 142 nmol CaNa₂EDTA and 112 µL cloudberry extract per gram oil.

### Example 25

### Powder comprising omega-3, ascorbic acid, EDTA and cloudberry extract (high concentrations)

To 20 g of the slurry prepared in example 21, 1150 µL 1 M ascorbic acid_{(aq)}, 568 µl 10 mM CaNa₂EDTA and 1442 µl of the cloudberry extract prepared in example 16 were added and stirred in using a glass rod. The product was dried by lyophilisation. The powder contained 351 µmol ascorbic acid, 554 nmol CaNa₂EDTA and 440 µL cloudberry extract per gram oil.

### Example 26 - comparative

### Slurry comprising omega-3 form EPA-ethyl ester and β-cyclodextrin

β-cyclodextrin (600 gram) and water (600 mL) were mixed in a kneader for 5 minutes. EPA-ethyl ester (400 gram) (Epax, Norway) was added and mixed in the kneader for 10 minutes. The oil:β-cyclodextrin ratio was 4:6.

### Example 27 - comparative

### Tablets comprising EPA-ethyl ester, β-cyclodextrin, Flowlac 100 and magnesium stearate

Tablets were from the powder prepared in example 26, Flowlac 100 (Meggle, Germany) and magnesium stearate in the ration 9.9:89.1:1. Tablets had a mean weight of 687 mg.

### Example 28

### Tablets compressing omega-3, β-cyclodextrin, Flowlac and magnesium stearate, coated with Kollicoat added ascorbic acid and EDTA

Kollicoat protect (10 gram) (BASF) was dissolved in a mixture of 1 M ascorbic acid_{(aq)} (40 gram) and 10 mM CaNa₂EDTA_{(aq)} (40 gram). Talc (6.3 gram) (Fluka) was suspended in water (20 gram). The two solutions were mixed and applied as a coating to tablets prepared in example 20 using a Drum Coater (Glatt, Germany).

### Example 29

### Accelerated stability study of powders with oxygen exposure at 37°C for up to 10 weeks

Powders described in examples 2-9 were stored at 37°C in plastic containers permeable to oxygen. Samples were collected on weeks 0, 2, 4, 7 and 10 and analysed for primary (PV = peroxide value) and secondary (AC = alkal count) oxidation products by PeroxySafe™ test kit and AlkalSafe™ test kit, respectively. Totox was calculated with the formula [Totox = (2×PV) + (AC/10)]. Results are listed in table 1.

**Table 1: Results of stability study of powders in examples 2-9**

| **Example** | **Totox per kg oil** | | | | |
|---|---|---|---|---|---|
| | **Week 0** | **Week 2** | **Week 4** | **Week 7** | **Week 10** |
| 2 | 8.9 | 149 | 373 | | |
| 3 | 6.4 | 425 | 2286 | | |
| 4 | 3.7 | 6.4 | 7.4 | 30 | 92 |
| 5 | 4.3 | 25 | 40 | 130 | |
| 6 | 9.2 | 8.5 | 4.1 | 7.4 | 23 |
| 7 | 3.1 | 65 | 447 | | |
| 8 | 8 | 279 | 3103 | | |
| 9 | 3.8 | 7.9 | 41 | 140 | |

### Example 30

### Accelerated stability study of powders with oxygen exposure at ambient temperature and 37°C for one week

Powders described in examples 11-12 were divided between plastic containers permeable to oxygen and light and plastic containers permeable to oxygen but not light. The light permeable containers were stored at 37°C and the light impermeable containers were stored at ambient temperature. Samples were collected weekly for three weeks and analysed for primary (PV = peroxide value) and secondary (AC = alkal count) oxidation products by PeroxySafe™ test kit and AlkalSafe™ test kit, respectively. Additional samples were collected after 6 weeks storage for the samples stored at room temperature. Totox was calculated with the formula [Totox = (2×PV) + (AC/10)]. Results are listed in table 2.

**Table 2: Results of stability study of powders in examples 11-12**

| **Example** | **Storage temperature** | **Totox per kg oil** | | |
|---|---|---|---|---|
| | | **Week 0** | **Week 3** | **Week 6** |
| 11 | 37°C | 17 | 84 | |
| 12 | 37°C | 11 | 22 | |
| 11 | Ambient | 17 | 24 | 74 |
| 12 | Ambient | 11 | 17 | 18 |

### Example 31

### Accelerated stability study of powders, tablets and oils with oxygen exposure at 37°C for one week

Powders described in examples 15 - 20were stored at 37°C in plastic containers permeable to oxygen for two weeks. Samples were collected at the start and after one weeks storage and analysed for primary oxidation products (PV = peroxide value) by PeroxySafe™ test kit. Results are listed in table 5.

**Table 5: Results of stability study of powders in examples 15 - 20**

| **Example** | **Matrix** | **Totox per kg oil** | |
|---|---|---|---|
| | | **Week** 0 | **Week 2** |
| 15 | Powder | 1.8 | 156 |
| 16 | Powder | 1.4 | 145 |
| 17 | Powder | 3.2 | 9.8 |
| 18 | Powder | 2.7 | 18 |
| 19 | Powder | 2.5 | 8.5 |
| 20 | Powder | 2.0 | 3.0 |

### Example 32

### Accelerated stability study of powders with oxygen exposure at 37°C for up to 10 weeks

Powders described in examples 22 - 25 were stored at 37°C in plastic containers permeable to oxygen. Samples were collected on weeks 0, 1, 2 and 4 and analysed for primary (PV = peroxide value) and secondary (AC = alkal count) oxidation products by PeroxySafe™ test kit and AlkalSafe™ test kit, respectively. Totox was calculated with the formula [Totox = (2×PV) + (AC/10)]. Results are listed in table 6.

**Table 6: Results of stability study of powders in examples 22-25**

| **Example** | **Totox per kg oil** | | | |
|---|---|---|---|---|
| | **Week 0** | **Week 1** | **Week 2** | **Week 4** |
| 22 | 8.8 | 8.4 | 11 | 421 |
| 23 | 5.6 | 51 | 556 | 3611 |
| 24 | 7.3 | 6.8 | 62 | 2611 |
| 25 | 4.2 | 10 | 15 | 183 |

### Example 33

### Powder comprising beta-cyclodextrin, high concentrate omega-3 triacylglycerol, ascorbic acid and EDTA

β-cyclodextrin (4.2 kg) and water (10.32 L) and an aquatic solution containing ascorbic acid at a concentration of 1 M and CaNa2EDTA at 10 mM (180 mL) a were mixed in a kneader for for 10 minutes. High concentrate omega-3 triacylglycerol (1.8 kg) (GC Rieber oil, Norway) was added and mixed in the kneader for 90 minutes. The slurry was dried by spray granulation. The oil:β-cyclodextrin ratio was 3:7.

### Example 34

### Powder comprising beta-cyclodextrin, EPA ethyl ester, ascorbic acid and EDTA

β-cyclodextrin (7 kg) and water (15 L) and an aquatic solution containing ascorbic acid at a concentration of 1 M and CaNa2EDTA at 10 mM (300 mL) a were mixed in a reactor for 60 minutes. EPA-ethyl ester (3 kg) (Epax, Norway) was added and mixed in the kneader for 90 minutes. The slurry was dried by spray granulation. The oil:β-cyclodextrin ratio was 3:7.

### Example 35

### Chewable capsules comprising high concentrate omega-3 tricaylglycerol powder, ascorbic acid and EDTA at high concentration

10 g gelatin was dissolved in 40 mL of a 1M ascorbic acid + 10 mM CaNa2EDTA solution and stirred at 60°C for 60 minutes. After cooling to about 40°C, the gel was mixed with 50 g high concentrate omega-3 triacylglycerol powder (example 33) and poured into portion sized molds.

### Example 36

### Chewable capsules comprising high concentrate omega-3 tricaylglycerol powder, ascorbic acid and EDTA at low concentration

10 g gelatin was dissolved in3 9.6 mL water and stirred at 60°C for 45 minutes. After cooling to about 40°C, 400 µl of a 1M ascorbic acid + 10 mM CaNa2EDTA solution was added, and the gel was mixed with 50 g high concentrate omega-3 triacylglycerol powder (example 33) and poured into portion sized molds.

### Example 37

### Chewable capsules comprising spray EPA ethyl ester powder, ascorbic acid and EDTA at high concentration

10 g gelatin was dissolved in 40 mL of a 1M ascorbic acid + 10 mM CaNa2EDTA solution and stirred at 60°C for 60 minutes. After cooling to about 40°C, the gel was mixed with 50 g EPA ethyl ester powder (example 34) and poured into portion sized molds.

### Example 38

### Chewable capsules comprising EPA ethyl ester powder, ascorbic acid and EDTA at low concentration

10 g gelatin was dissolved in 3 9.6 mL water and stirred at 60°C for 45 minutes. After cooling to about 40°C, 400 µl of a 1M ascorbic acid + 10 mM CaNa2EDTA solution was added, and the gel was mixed with 50 g EPA ethyl ester powder (example 34) and poured into portion sized molds.

### Example 39

### Portion size biscuits comprising high concentrate omega-3 tricaylglycerol powder, ascorbic acid and EDTA at high concentration

Two oatmeal biscuits (30 g) was crushed and mixed with high concentrate omega-3 triacylglycerol powder (example 53) in a mortar. 10 ml of a solution of ascorbic acid (1M) and CaNa2EDTA (10 mM) was mixed in. The paste was formed into portion sized biscuits and dried by lyophilisation.

### Example 40

### Portion size biscuits comprising high concentrate omega-3 tricaylglycerol powder, ascorbic acid and EDTA at low concentration

Two oatmeal biscuits (30 g) was crushed and mixed with high concentrate omega-3 triacylglycerol powder (example 33) in a mortar. 10 ml of a solution of ascorbic acid (10 mM) and CaNa2EDTA (100 µM) was mixed in. The paste was formed into portion sized biscuits and dried by lyophilisation.

### Example 41

### Portion size biscuits comprising EPA ethyl ester powder, ascorbic acid and EDTA at high concentration

Two oatmeal biscuits (30 g) was crushed and mixed with EPA ethyl ester powder (example 34) in a mortar. 10 ml of a solution of ascorbic acid (1M) and CaNa2EDTA (10 mM) was mixed in. The paste was formed into portion sized biscuits and dried by lyophilisation.

### Example 42

### Portion size biscuits comprising EPA ethyl ester powder, ascorbic acid and EDTA at low concentration

Two oatmeal biscuits (30 g) was crushed and mixed with EPA ethyl ester powder (example 34) in a mortar. 10 ml of a solution of ascorbic acid (10 mM) and CaNa2EDTA (100 µM) was mixed in. The paste was formed into portion sized biscuits and dried by lyophilisation.

### Example 43

### Effervescent tablets comprising high concentrate omega-3 tricaylglycerol powder, ascorbic acid and EDTA

High concentrate omega-3 triacylglycerol powder (example 53) (6g) was mixed with citric acid (2.5 g), sodium bicarbonate (2 g), polyvinylpyrrolidone (230 mg) and talc (100 mg) in a mortar. 4 tablets at 2.7 g were made from the mixture.

### Example 44

### Effervescent tablets comprising high EPA ethyl ester powder, ascorbic acid and EDTA

EPA ethyl ester powder (example 54) (6g) was mixed with citric acid (2.5 g), sodium bicarbonate (2 g), polyvinylpyrrolidone (230 mg) and talc (500 mg) in a mortar. 4 tablets at 2.8 g were made from the mixture.

### Example 45

### Effervescent tablets comprising high concentrate omega-3 tricaylglycerol powder, ascorbic acid and EDTA

High concentrate omega-3 triacylglycerol powder (example 53) (3g) was mixed with citric acid (5 g), sodium bicarbonate (4 g), polyvinylpyrrolidone (230 mg) and talc (500 mg) in a mortar. 5 tablets at 2.5 g were made from the mixture.

### Example 46

### Effervescent tablets comprising high EPA ethyl ester powder, ascorbic acid and EDTA

EPA ethyl ester powder (example 54) (3g) was mixed with citric acid (5 g), sodium bicarbonate (4 g), polyvinylpyrrolidone (230 mg) and talc (500 mg) in a mortar. 5 tablets á 2.5 g were made from the mixture.

### Example 47

### Powder comprising beta-cyclodextrin, EPA ethyl ester, ascorbic acid and EDTA

β-cyclodextrin (900 g) and water (1070 mL) and an aquatic solution containing ascorbic acid at a concentration of 1 M and CaNa2EDTA at 10 mM (30 mL) a were mixed in a reactor for 10 minutes. EPA-ethyl ester (300 g) (Epax, Norway) was added and mixed in the kneader for 10 minutes. The slurry was dried by spray granulation. The oil:β-cyclodextrin ratio was 25:75.

### Example 48

### Tablets comprising EPA ethyl ester powder, ascorbic acid and EDTA

EPA ethyl ester powder (example 54) (98.75 % w/w), talc (1% w/w) and magnesium stearate (0.25 % w/w) were mixed. Tablets á 800 mg were made from the mixture.

### Example 49

### Chewable capsules comprising high concentrate omega-3 triacylglycerol emulsion with ascorbic acid and EDTA at low concentration

10 g gelatin was dissolved in 40 mL of a 1M ascorbic acid + 10 mM CaNa₂EDTA solution and stirred at 60°C for 60 minutes. 50 g sorbitol was added and dissolved under stirring for 60 minutes. After cooling to about 40°C, the gel was mixed with 50 g high concentrate omega-3 triacylglycerol oil (GC Rieber Oil) and poured into portion sized molds.

### Example 50

### Chewable capsules comprising high concentrate omega-3 triacylglycerol emulsion with ascorbic acid and EDTA at high concentration

10 g gelatin was dissolved in3 9.6 mL water and stirred at 60°C for 60 minutes. 50 g sorbitol was added and dissolved under stirring for 60 minutes. After cooling to about 40°C, 400 µl of a 1M ascorbic acid + 10 mM CaNa₂EDTA solution was added, and the gel was mixed with 50 g high concentrate omega-3 triacylglycerol oil (GC Rieber Oil) and poured into portion sized molds.

### Example 51

### Effervescent tablets comprising high concentrate omega-3 tricaylglycerol powder, ascorbic acid and EDTA

High concentrate omega-3 triacylglycerol powder (example 33) (6g) was mixed with citric acid (2.5 g), sodium bicarbonate (2 g), sorbitol (1g), polyvinylpyrrolidone (230 mg) and talc (100 mg) in a mortar. 4 tablets at 2.8 g were made from the mixture.

### Example 52

### Effervescent tablets comprising high EPA ethyl ester powder, ascorbic acid and EDTA

EPA ethyl ester powder (example 34) (6g) was mixed with citric acid (2.5 g), sodium bicarbonate (2 g), sorbitol (1 g), polyvinylpyrrolidone (230 mg) and talc (500 mg) in a mortar. 4 tablets at 2.8 g were made from the mixture.

### Example 53

### Effervescent tablets comprising high concentrate omega-3 tricaylglycerol powder, ascorbic acid and EDTA

High concentrate omega-3 triacylglycerol powder (example 33) (3g) was mixed with citric acid (5 g), sodium bicarbonate (4 g), sorbitol (2g), polyvinylpyrrolidone (230 mg) and talc (500 mg) in a mortar. 5 tablets at 2.8 g were made from the mixture.

### Example 54

### Effervescent tablets comprising high EPA ethyl ester powder, ascorbic acid and EDTA

EPA ethyl ester powder (example 34) (3g) was mixed with citric acid (5 g), sodium bicarbonate (4 g), sorbitol (2g), polyvinylpyrrolidone (230 mg) and talc (500 mg) in a mortar. 5 tablets at 2.8 g were made from the mixture.

### REFERENCES

Han, D., Yi, O.S., Shin, H.K. (1990): "Antioxidative effect of ascorbic acid solubilized in oils via reversed micelles", Journal of Food Science, 55, 247-249.
Nishina, A. (1991): "Antioxidant effects of tocopherols and L-ascorbic acid on ethyl eicosapentanoate and methyl linoleate", Agricultural and Biological Chemistry, 55, 1665-1667.
Olsen, E., Vogt, G., Saarem, K., Greibrokk, T, Nilsson, A. (2005): "Autooxidation of cod liver oil with tocopherol and ascorbyl palmitate", Journal of the American Oil Chemists' Society, 82, 97-103.
Let, M.B., Jacobsen, C., Pham, K.A., Meyer, A.S. (2005): "Protection against oxidation of fish-oil-enriched milk emulsions through addition of rapeseed oil or antioxidants", Journal of Agricultural and Food Chemistry, 53, 5429-5437.
Jacobsen, C., Let, M.B., Nielsen, N.S, Meyer, A.S. (2008): "Antioxidant strategies for preventing oxidative flavor deterioration in foods enriched with n-3 polyunsaturated lipids: a comparative evaluation", Trends in Food Science and Technology, 19, 76-93.
Frankel, E.N., Satué-Gracia, T., Meyer, A.S., German, J.B. (2002): "Oxidative stability of fish and algae oils containing long-chain polyunsaturated fatty acids in bulk and in oil-in-water emulsions", Journal of Agricultural and Food Chemistry, 50, 2094-2099.
Nielsen, N.S, Petersen, A., Meyer, A.S., Timm-Heinrich, M., Jacobsen, (2004): "Effects of lactoferrin, phytic acid, and EDTA in oxidation in two food emulsions enriched with long-chain polyunsaturated fatty acids", Journal of Agricultural and Food Chemistry, 52, 7690-7699.
Let, M.B., Jacobsen, C., Meyer, A.S. (2007): "Ascorbyl palmitate, γ-tocopherol, and EDTA affect lipid oxidation in fish oil enriched salad dressing differently", Journal of Agricultural and Food Chemistry, 55, 2369-2375.
Olsen, E., Veberg, A., Vogt, G., Tomic, O., Kirkhus, B., Ekeberg, D, Nilsson, A. (2006): "Analysis of early lipid oxidation in salmon pâté with cod liver oil and antioxidants", Journal of Food Science, 71, S284-S292.
Haak, L., Raes, K., De Smet, S. (2009): "Effect of plant phenolics, tocopherol and ascorbic acid on oxidative stability of pork patties", Journal of the Science of Food and Agriculture, 89, 1360-1365.

## Claims

1. A stable powder comprising a fatty acid compound, carrier, ascorbic acid at a concentration of 2 mmol - 500 mmol per kg powder and a metal chelator which is EDTA at a concentration of 10 micromol - 4 mmol per kg powder, said powder being capable of maintaining a Totox/kg oil of less than 100 for 10, 20, 30, 40 or 50 weeks at room temperature in the presence of oxygen and the absence of light.

2. The stable powder of claim 1, said powder being capable of maintaining a Totox/kg oil of less than 50 for 10, 20, 30, 40 or 50 weeks at room temperature in the presence of oxygen and the absence of light.

3. The stable powder of claim 2, said powder being capable of maintaining a Totox/kg oil of less than 25 for 10, 20, 30, 40 or 50 weeks at room temperature in the presence of oxygen and the absence of light.

4. The stable powder of any of claims 1 to 3, wherein said carrier is selected from the group consisting of cyclodextrin, microcrystalline cellulose, and combinations thereof.

5. The stable powder of Claim 4, wherein said cyclodextrin is beta-cyclodextrin.

6. The stable powder of Claims 3 or 4, wherein said fatty acid compound is complexed with said cyclodextrin.

7. The stable powder of and of Claims 1 to 6, wherein said fatty acid compound is selected from the group consisting of free fatty acids, triglycerides, fatty acid esters, phospholipids and combinations thereof, said fatty acid compound preferably comprising fatty acid moieties selected from the group consisting of EPA, DHA, and conjugated linoleic acid, said fatty acid compound preferably comprising a fatty acid compound preparation selected from the group consisting of fish oil, salmon oil, cod liver oil, omega-3 concentrate, krill oil, and algal oil.

8. The stable powder of any of Claims 1 to 7, wherein said powder comprises 10 percent to 50 percent fatty acid compound on w/w basis per total mass of powder.

9. The stable powder of any of Claims 1 to 8, further comprising a gelling agent.

10. A pharmaceutical or nutraceutical oral delivery vehicle for oral administration comprising the stable powder of any of Claims 1 to 9.

11. Oral delivery vehicle according to claim 10, being in the form of a tablet or a capsule.

12. The oral delivery vehicle of Claim 11, further comprising a functional coating comprising a coating material and at least one functional material.

13. The oral delivery vehicle of Claim 12, wherein said coating material is selected from the group consisting of polyvinyl alcohol, polyvinyl alcohol copolymers, hydroxypropylmethyl cellulose, ethylcellulose, methylcllulose, hypomellose, hydroxyethylcellulose, polyvinylpyrrolidine, polyacrylates, polyethyleneglycol, sugar, gelatin, chitin, chitosan, titanium oxide, pH sensitive polymers and cellulose acetate phthalate.

14. The oral delivery vehicle of Claim 12 or 13, wherein said functional material is selected from the group consisting of a specifically degradable material, a light absorbing material, a material that enhances hydrophobic stability, and a material that enhances oxidative stability, and combinations thereof.

## Patentansprüche

1. Ein stabiles Pulver, das eine Fettsäureverbindung, einen Träger, Ascorbinsäure mit einer Konzentration von 2 mmol - 500 mmol pro kg Pulver und einen Metallchelatbildner, der EDTA ist, mit einer Konzentration von 10 µmol - 4 mmol pro kg Pulver umfasst, wobei dieses Pulver in der Lage ist, 10, 20, 30, 40 oder 50 Wochen lang eine Totox/kg Öl von kleiner als 100 bei Raumtemperatur und Anwesenheit von Sauerstoff und Abwesenheit von Licht aufrechtzuerhalten.

2. Das stabile Pulver nach Anspruch 1, wobei dieses Pulver in der Lage ist, 10, 20, 30, 40 oder 50 Wochen lang eine Totox/kg Öl von kleiner als 50 bei Raumtemperatur und Anwesenheit von Sauerstoff und Abwesenheit von Licht aufrechtzuerhalten.

3. Das stabile Pulver nach Anspruch 2, wobei dieses Pulver in der Lage ist, 10, 20, 30, 40 oder 50 Wochen lang eine Totox/kg Öl von kleiner als 25 bei Raumtemperatur und Anwesenheit von Sauerstoff und Abwesenheit von Licht aufrechtzuerhalten.

4. Das stabile Pulver nach einem der Ansprüche 1 bis 3, wobei der Träger aus der Gruppe ausgewählt ist, die aus Cyclodextrin, mikrokristalliner Cellulose und Kombinationen davon besteht.

5. Das stabile Pulver nach Anspruch 4, wobei das Cyclodextrin β-Cyclodextrin ist.

6. Das stabile Pulver nach Anspruch 3 oder 4, wobei die Fettsäureverbindung mit dem Cyclodextrin komplexiert ist.

7. Das stabile Pulver nach einem der Ansprüche 1 bis 6, wobei die Fettsäureverbindung aus der Gruppe ausgewählt ist, die aus freien Fettsäuren, Triglyceriden, Fettsäureestern, Phospholipiden und Kombinationen davon besteht, wobei die Fettsäureverbindung vorzugsweise Fettsäurereste, die aus der Gruppe ausgewählt sind, die aus EPA, DHA und konjugierter Linolsäure besteht, umfasst, wobei die Fettsäureverbindung vorzugsweise ein Fettsäureverbindungspräparat, das aus der Gruppe ausgewählt ist, die aus Fischöl, Lachsöl, Lebertran, Omega-3-Konzentrat, Krillöl und Algenöl besteht, umfasst.

8. Das stabile Pulver nach einem der Ansprüche 1 bis 7, wobei dieses Pulver 10 Prozent bis 50 Prozent Fettsäureverbindung auf der Basis Gew./Gew. pro Gesamtmasse Pulver umfasst.

9. Das stabile Pulver nach einem der Ansprüche 1 bis 8, das weiterhin ein Geliermittel umfasst.

10. Ein pharmazeutisches oder nutrazeutisches Oralverabreichungsvehikel bei oraler Gabe, welches das stabile Pulver nach einem der Ansprüche 1 bis 9 umfasst.

11. Oralverabreichungsvehikel nach Anspruch 10, das in Form einer Tablette oder Kapsel vorliegt.

12. Das Oralverabreichungsvehikel nach Anspruch 11, das ferner eine funktionelle Beschichtung umfasst, die ein Beschichtungsmaterial und mindestens ein funktionelles Material umfasst.

13. Das Oralverabreichungsvehikel nach Anspruch 12, wobei das Beschichtungsmaterial aus der Gruppe ausgewählt ist, die aus Polyvinylalkohol, Polyvinylalkoholcopolymeren, Hydroxypropylmethylcellulose, Ethylcellulose, Methylcellulose, Hypomellose, Hydroxyethylcellulose, Polyvinylpyrrolidin, Polyacrylaten, Polyethylenglykol, Zucker, Gelatine, Chitin, Chitosan, Titanoxid, pH-empfindlichen Polymeren und Celluloseacetatphthalat besteht.

14. Das Oralverabreichungsvehikel nach Anspruch 12 oder 13, wobei das funktionelle Material aus der Gruppe ausgewählt ist, die aus spezifisch abbaubarem Material, einem Licht absorbierenden Material, einem Material, das die hydrophobe Stabilität verbessert, und einem Material, durch welches die oxidative Stabilität verbessern, und Kombinationen davon besteht.

## Revendications

1. Poudre stable comprenant un composé d'acide gras, un support, de l'acide ascorbique à une concentration allant de 2 mmol à 500 mmol par kg de poudre et un chélateur de métaux qui est de l'EDTA à une concentration allant de 10 micromol à 4 mmol par kg de poudre, ladite poudre étant capable de maintenir un indice d'oxydation totale (Totox)/kg d'huile inférieur à 100 pendant 10, 20, 30, 40 ou 50 semaines à température ambiante en présence de l'oxygène et en l'absence de lumière.

2. Poudre stable de la revendication 1, ladite poudre étant capable de maintenir un Totox/kg d'huile inférieur à 50 pendant 10, 20, 30, 40 ou 50 semaines à température ambiante en présence de l'oxygène et en l'absence de lumière.

3. Poudre stable de la revendication 2, ladite poudre étant capable de maintenir un Totox/kg d'huile inférieur à 25 pendant 10, 20, 30, 40 ou 50 semaines à température ambiante en présence de l'oxygène et en l'absence de lumière.

4. Poudre stable selon l'une des revendications 1 à 3, dans laquelle ledit support est choisi dans le groupe constitué par la cyclodextrine, la cellulose microcristalline et des combinaisons de celles-ci.

5. Poudre stable de la revendication 4, dans laquelle ladite cyclodextrine est la bêta-cyclodextrine.

6. Poudre stable de la revendication 3 ou 4, dans laquelle ledit composé d'acide gras forme un complexe avec ladite cyclodextrine.

7. Poudre stable des revendications 1 à 6, dans laquelle ledit composé d'acide gras est choisi dans le groupe constitué par des acides gras libres, des triglycérides, des esters d'acides gras, des phospholipides et des combinaisons de ceux-ci, ledit composé d'acide gras comprenant de préférence des fractions d'acide gras sélectionnées dans le groupe constitué par l'EPA, le DHA et l'acide linoléique conjugué, ledit composé d'acide gras comprenant de préférence une préparation de composé d'acide gras choisie dans le groupe constitué par l'huile de poisson, l'huile de saumon, l'huile de foie de morue, le concentré d'oméga-3, l'huile de krill et l'huile d'algues.

8. Poudre stable de l'une des revendications 1 à 7, dans laquelle ladite poudre comprend 10 pour cent à 50 pour cent de composé d'acide gras en poids/poids par masse totale de poudre.

9. Poudre stable de l'une des revendications 1 à 8, comprenant en outre un agent gélifiant.

10. Véhicule d'administration orale pharmaceutique ou nutraceutique pour une administration orale comprenant la poudre stable de l'une des revendications 1 à 9.

11. Véhicule d'administration orale selon la revendication 10, se présentant sous la forme d'un comprimé ou d'une capsule.

12. Véhicule d'administration orale de la revendication 11, comprenant en outre un revêtement fonctionnel qui comprend un matériau de revêtement et au moins un matériau fonctionnel.

13. Véhicule d'administration orale de la revendication 12, dans lequel ledit matériau de revêtement est choisi dans le groupe constitué par l'alcool polyvinylique, des copolymères d'alcool polyvinylique, l'hydroxypropylméthylcellulose, l'éthylcellulose, la méthylcellulose, l'hypromellose, l'hydroxyéthylcellulose, la polyvinylpyrrolidine, des polyacrylates, le polyéthylèneglycol, le sucre, la gélatine, la chitine, le chitosane, l'oxyde de titane, des polymères sensibles au pH et l'acétophtalate de cellulose.

14. Véhicule d'administration orale de la revendication 12 ou 13, dans lequel ledit matériau fonctionnel est choisi dans le groupe constitué par un matériau spécifiquement dégradable, un matériau absorbant la lumière, un matériau améliorant la stabilité hydrophobe et un matériau améliorant la stabilité à l'oxydation et des combinaisons de ceux-ci.
